# EUROPEAN PATENT SPECIFICATION

(11) **EP 4 241 719 B1**
(45) Date of publication and mention of the grant of the patent: **30.04.2025**
(21) Application number: 23177759.0
(22) Date of filing: 13.07.2017
(51) Int. Cl.: A61B 18/12, A61B 17/32, A61B 18/14, A61B 90/00

(54) **MEDICAL DEVICE ENERGY SOURCE AND MEDICAL DEVICE SYSTEM**
ENERGIEQUELLE FÜR MEDIZINISCHES GERÄT UND MEDIZINISCHES GERÄTESYSTEM
SOURCE D'ENERGIE POUR UN OUTIL MEDICAL ET SYSTEM D'UN OUTIL MEDICAL

(30) Priority: 15.07.2016 US 201615212141
(43) Date of publication of application: 13.09.2023
(62) Divisional of application: 17743163.2
(73) Proprietor: Ethicon LLC, 00969 Guaynabo (PR)
(72) Inventor: DICKERSON, Benjamin D., Cincinnati, 45242 (US)
(74) Representative: Carpmaels & Ransford LLP

(56) References cited:
- US-A1- 2004 116 952
- US-A1- 2009 043 293
- US-A1- 2009 065 565
- US-A1- 2014 272 771
- US-A1- 2015 257 817
- US-B1- 6 611 793

## Description

### BACKGROUND

Electrosurgical devices are used in many surgical procedures which may include removing, shrinking, or sealing tissues as part of the therapeutic process. In some examples, electrosurgical devices may apply electrical energy directly to tissue in order to effect the surgical treatment. Alternatively, electrosurgical devices may use the electrical energy as a source of power for other modes of surgical treatment, for example to generate ultrasonic energy which may then be applied to the tissues. An electrosurgical device may comprise an instrument having a distally-mounted end effector comprising components designed to introduce the therapeutic energy into the tissue being treated. Such end effectors may consist of two or more jaws in which at least one of the jaws is moveable from a position spaced apart from the opposing jaw for receiving tissues to a position in which the space between the jaws is less than that of the first position. Movement of the moveable jaw may compress the tissue held between. The therapeutic energy delivered by components of the end effector, in combination with the compression achieved by the jaw movement, may form hemostatic seals within the tissue and/or between tissues and thus may be particularly useful for sealing blood vessels, for example. The end effector of an electrosurgical device may also comprise a cutting member that is movable relative to the tissue and the jaws to transect the tissue.

In some electrosurgical devices, electrical energy may be transmitted to the instrument by a generator and applied directly by the electrosurgical device to the tissue under treatment. In some examples, the electrical energy may be in the form of radio frequency ("RF") energy. The electrical energy may be in the form of radio frequency ("RF") energy that may be in a frequency range described in EN 60601-2-2:2009+A11:20. In some applications, the applied energy may have a frequency restricted to less than 5MHz. Typically, frequencies above 5 MHz are not used in order to minimize the problems associated with high frequency leakage currents. It is generally recognized that 10 mA is a lower threshold of thermal effects on tissue. RF energy may be supplied by a power source and introduced into tissue compressed between the two or more jaws. Such RF energy may cause ionic agitation in the tissue, in effect producing resistive heating, and thereby increasing the temperature of the tissue. Increased temperature of the tissue may lead to tissue cauterization. In some surgical procedures, RF energy may be useful for removing, shrinking, or sculpting soft tissue while simultaneously sealing blood vessels.

Other electrosurgical devices may use the electrical energy as a source of power for other modes of surgical treatment, for example to generate ultrasonic energy which may then be applied to the tissues. Ultrasonic surgical instruments can be used for the safe and effective treatment of many medical conditions. Generally, ultrasonic surgical instruments can be used to cut and/or coagulate organic tissue, for example, using energy in the form of ultrasonic vibrations, i.e., mechanical vibrations transmitted to a surgical end-effector at ultrasonic frequencies. These ultrasonic vibrations, when transmitted to organic tissue at suitable energy levels and using a suitable end-effector, may be used to cut and/or coagulate the tissue. Such instruments may be used for open procedures or minimally invasive procedures, such as endoscopic or laparoscopic procedures, for example, in which the end-effector of the medical device is passed through a trocar to reach a surgical site.

It may be recognized that proper control of the electrical energy supplied to an electrosurgical device is critical for safe and effective operation of the device. It is therefore desirable for a medical device energy source to supply an appropriate amount of electrical energy to an electrosurgical device to promote a safe and effective therapeutic outcome. For example, an appropriate amount of electrical energy may be dependent on the type of electrosurgical device and its usage history.
US2015/257817 discloses an electrosurgical apparatus including an automatic applicator identifier used to communicate between an applicator and a generator unit, automatically presetting various values. These values may be stored in a one-wire serial memory storage device located in the applicator or connector coupled to the applicator. Communication from the applicator to the generator unit of these values is affected by a one-wire serial communication protocol. This information can be transferred over a direct electrical path through the connector that attaches that applicator to the generator unit, or instead by a wireless link.
US2014/0272771 discloses a medical treatment apparatus that includes a power and control (PAC) device. The PAC device provides electrical power through a cable to a laser handpiece assembly to electrically power a laser source within the handpiece assembly. The PAC device controls operation of the handpiece assembly and detects an identification of the handpiece assembly. The PAC device also monitors data relating to operation of the handpiece assembly. The PAC device uploads, through a communication network to a user assistance center remote from the PAC device, the handpiece assembly identification and monitored data.

### SUMMARY

The invention is defined in independent claims 1 and 7. Further embodiments are described in the dependent claims.
In one aspect, a medical device energy source may be composed of an energy source, an energy source power interface configured to deliver electrical energy from the energy source, and an energy source computing device. The energy source computing device may further be composed of an energy source processor unit, an energy source memory storage component in operative communication with the energy source processor unit, an energy source network communication interface in operative communication with the energy source processor unit, and an energy source data interface in operative communication with the energy source processor unit. The energy source computing device may be configured to control a function of the energy source. Further, the energy source memory storage component may include instructions that, when executed by the energy source processor unit, may cause the energy source computing device to receive an identifier code via the energy source data interface, receive a plurality of medical device identity codes via the energy source network communication interface, compare the identifier code with each of the plurality of medical device identity codes, and control the
function of the energy source based on the comparison of the identifier code with each of the plurality of medical device identity codes.

In one aspect of the medical device energy source, the identifier code may be composed of two identifier strings, each of which may include a string of processor readable characters.

In one aspect of the medical device energy source, the instructions that cause the energy source computing device to compare the identifier code with each of the plurality of medical device identity codes may include instructions that cause the energy source computing device to compare each of two identifier strings with each of two identity strings comprising each of the medical device identity codes.

In one aspect of the medical device energy source, the energy source memory storage component may include instructions that, when executed by the energy source processor unit, further cause the energy source computing device to receive, via the energy source network communication interface, a plurality of medical device status indicators, in which each medical device status indicator corresponds to each of the plurality of medical device identity codes.

In one aspect of the medical device energy source, the instructions that cause the energy source computing device to control the function of the energy source further comprise instructions that cause the energy source computing device to control the function of the energy source base on the medical device status indicators corresponding to a medical device identity code equal to the identifier code.

In one aspect of the medical device energy source, the energy source memory storage component may include instructions that, when executed by the energy source processor unit, further cause the energy source computing device to retain, in the energy source memory storage component, an energizer value corresponding to an amount of energy supplied by the energy source, an energizer time value corresponding to a length of time during which the energy source supplies an amount of energy, an energizer number corresponding to a number of times the energy source supplies an amount of energy, or combinations thereof.

In one aspect, the medical device energy source may further include a user display in operative communication with the energy source processor unit.

In one aspect, a medical device system may be composed of a medical device, a medical device energy source, and a medical device network server. The medical device may be composed of a device memory storage component configured to store an identifier code; a device data interface in operative connection with the memory storage component; and a device power interface configured to receive electric power from an energy source. The medical device energy source may be composed of the energy source, an energy source power interface in operative communication with the device power interface and configured to deliver electrical energy from the energy source to the medical device, and an energy source computing device. The energy source computing device may further be composed of an energy source processor unit, an energy source memory storage component in operative communication with the energy source processor unit, an energy source network communication interface in operative communication with the energy source processor unit and configured to transmit data to and receive data from a communication network, and an energy source data interface in operative connection with the device data interface, in which the energy source computing device may be configured to control a function of the energy source. The medical device network server may be composed of a network server processor unit, a network server memory storage component in operative communication with the network server processor unit and configured to store a medical device database comprising a plurality of medical device identity codes and corresponding medical device status indicators, and a network server communication interface in operative communication with the network server processor unit and configured to transmit data to and receive data from at least one medical device power source via the communication network. In this aspect, the energy source memory storage component may include instructions that, when executed by the energy source processor unit, cause the energy source computing device to receive, from the device memory storage component, the identifier code, receive, from the network server memory storage component, the plurality of medical device identity codes from the medical device database, compare the identifier code with each of the plurality of medical device identity codes, and control the function of the energy source based on the comparison of the at least one identifier code with the plurality of medical device identity codes.

In one aspect of the medical device system, the identifier code may be composed of two identifier strings, each of which may include a string of processor readable characters. In this aspect, the instructions that cause the energy source computing device to compare the identifier code with each of the plurality of medical device identity codes includes instructions that cause the energy source computing device to compare each of the two identifier strings with each of two identity strings comprising each of the medical device identity codes.

In one aspect of the medical device system, the energy source memory storage component may include instructions that, when executed by the energy source processor unit, further cause the energy source computing device to receive, via the energy source network communication interface, a plurality of medical device status indicators, in which each of the plurality of medical device status indicators corresponds to each of the plurality of medical device identity codes. Further, the instructions that cause the energy source computing device to control the function of the energy source further include instructions that cause the energy source computing device to control the function of the energy source base on a medical device status indicator corresponding to a medical device identity code equal to the identifier code.

In one aspect of the medical device system, the energy source memory storage component may further include instructions that, when executed by the energy source processor unit, cause the energy source computing device to transmit, to the medical device network server, data to update a medical device status indicator corresponding to a medical device identity code equal to the identifier code.

In one aspect of the medical device system, the network server memory storage component may include instructions that, when executed by the network servicer processor unit, cause the network server processor unit to receive, from the medical device energy source, data to update a medical device status indicator corresponding to the medical device identity code equal to the identifier code and to update the status indicator in the data base corresponding to the medical device identity code equal to the identifier code.

In one aspect of the medical device system, the medical device data base may further include one or more additional indicators corresponding to each of the medical device identity codes in the medical device data base.

In one aspect of the medical device system, the energy source memory storage component may further include instructions that, when executed by the energy source processor unit, cause the energy source computing device to store in the energy source memory storage component an indicator of total medical device uses and a total amount of power supplied by the medical device energy source to the medical device over the total number of medical device uses. The energy source memory storage component may further include instructions that, when executed by the energy source processor unit, cause the energy source computing device to store in the energy source memory storage component an indicator, for each use of the total medical device uses, of an amount of power supplied by the medical device energy source to the medical device, and a length of time during which the medical device energy source supplies the amount of energy to the medical device.

In one aspect of the medical device system, the energy source memory storage component may further include instructions that, when executed by the energy source processor unit, cause the energy source computing device to receive, from the medical device network server, values of the one or more additional indicators corresponding to each of the medical device identity codes in the medical device data base and control the function of the energy source based on the value of the one or more of the additional indicators corresponding to the medical device identity code equal to the identifier code.

In one aspect of the medical device system, he network server memory storage component may include instructions that, when executed by the network servicer processor unit, cause the network server processor unit to receive, from the medical device energy source, values of the one or more additional indicators corresponding to each of the medical device identity codes in the medical device data base, and update the values of the one or more additional indicators corresponding to each of the medical device identity codes in the medical device database.

The invention is defined by the appended claims. No methods are claimed as such, although some methods described herein may nevertheless be useful for understanding the invention. In an aspect, a method (not claimed as such) of controlling a medical device may include receiving, by a medical device energy source via an energy source data interface in operative communication with an energy source processor unit, an identifier code from a medical device, storing, by the medical device energy source in an energy source memory storage component in operative communication with the energy source processor unit, the identifier code, receiving, by the medical device energy source via an energy source network communication interface in operative communication with the energy source processor unit, a plurality of medical device identity codes from a medical device network server, comparing, by the energy source processor unit, the identifier code with each of the plurality of medical device identity codes, controlling, by the energy source processor unit, an amount of energy delivered by the energy source via an energy source power interface to the medical device, based on the comparison between the identifier code and the plurality of medical device identity codes, and displaying, on a user display operatively controlled by an energy source computing device comprising the energy source processor unit, information corresponding to the amount of energy delivered by the energy source to the medical device.

In an aspect, the method (not claimed as such) may further include transmitting, by the medical device energy source to the medical device, control data to control at least one function of the medical device.

In an aspect, the method (not claimed as such) may further include receiving, by the medical device energy source via the energy source network communication interface, a plurality of medical device status indicators, wherein each of the plurality of medical device status indicators corresponds to each of the plurality of medical device identity codes.

In an aspect, the method (not claimed as such) may further include controlling, by the energy source processor unit, an amount of energy delivered by the energy source via the energy source power interface to the medical device, based on the medical device status indicator corresponding to a medical device identity code that is equal to the identifier code.

In an aspect, the method (not claimed as such) may further include transmitting, by the medical device energy source to the medical device, control data to control at least one function of the medical device based on the medical device status indicators corresponding to the medical device identity code that is equal to the identifier code.

### BRIEF DESCRIPTION OF THE FIGURES

The features of the various aspects are set forth with particularity in the appended claims. The various aspects, however, both as to organization and methods of operation, together with advantages thereof, may best be understood by reference to the following description, taken in conjunction with the accompanying drawings as follows:
FIG. 1A illustrates one form of a surgical system comprising a generator and various surgical instruments usable therewith.
FIG. 1B is a diagram of the ultrasonic surgical instrument of FIG. 1.
FIG. 1C is a diagram of the surgical system of FIG. 1.
FIG. 2 illustrates a block diagram of an example of a medical device system.
FIGS. 3A and 3B illustrate block diagrams of examples of medical devices which may be a component of the medical device system illustrated in FIG. 2.
FIGS. 4A and 4B illustrate block diagrams of examples of medical device energy sources which may be a component of the medical device system illustrated in FIG. 2.
FIG. 5 illustrates a block diagram of an example of a medical device network server which may be a component of the medical device system illustrated in FIG. 2.
FIGS. 6A-6D illustrate examples of data structures in a database which may be stored in a memory component of the medical device network server illustrated in FIG. 5.
FIGS. 7A and 7B are simplified flow charts illustrating programming a medical device for use in the medical device system illustrated in FIG. 2.
FIGS. 8A and 8B are simplified flow charts illustrating using a medical device which is part of the medical device system illustrated in FIG. 2.

### DETAILED DESCRIPTION

Reference will now be made in detail to several aspects, including example implementations of electrosurgical medical instruments for cutting and coagulating tissue. Wherever practicable similar or like reference numbers may be used in the figures and may indicate similar or like functionality. The figures depict examples of the disclosed surgical instruments and/or methods of use for purposes of illustration only. One skilled in the art will readily recognize from the following description that alternative examples of the structures and methods illustrated herein may be employed without departing from the principles described herein.

Various aspects of surgical instruments that utilize therapeutic and/or sub-therapeutic electrical energy to treat tissue or provide feedback to the generators (e.g., electrosurgical instruments). The various aspects are adapted for use in a manual or hand operated manner, although electrosurgical instruments may be utilized in robotic applications as well.

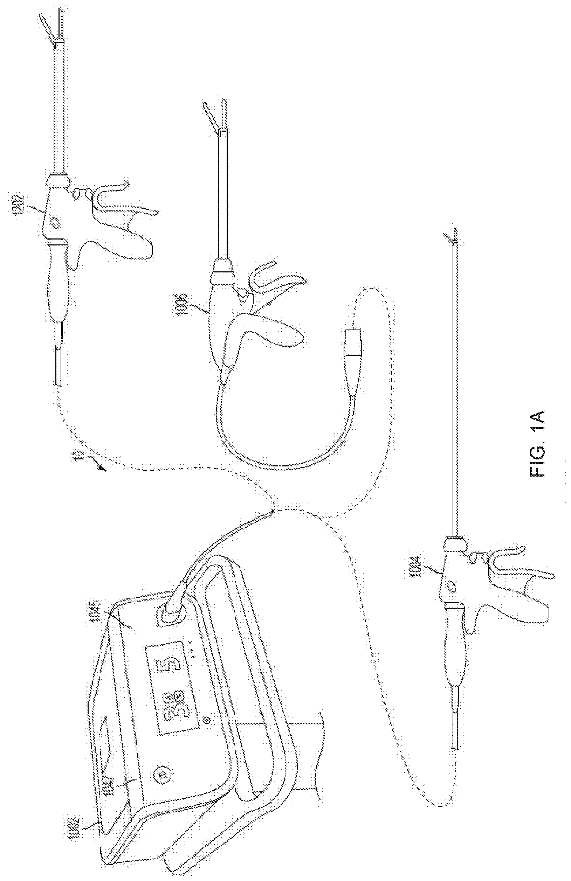

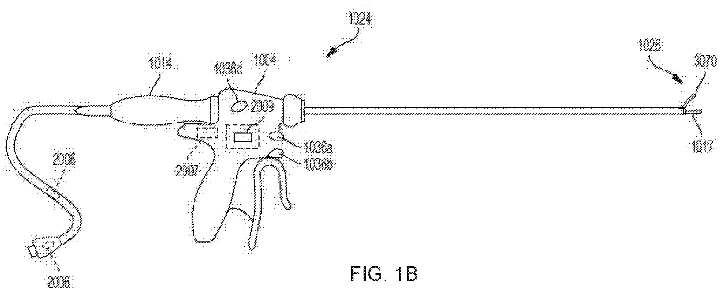

With reference to FIGS. 1A-1C, one form of a surgical system **10** including an ultrasonic surgical instrument is illustrated. FIG. 1A illustrates one form of a surgical system **10** comprising a generator **1002** and various surgical instruments **1004, 1006, 1202** usable therewith. FIG. 1B is a diagram of the ultrasonic surgical instrument **1004** of FIG. 1A. With reference to both FIGS. 1A and 1B, the generator **1002** is configurable for use with a variety of surgical devices. According to various forms, the generator **1002** may be configurable for use with different surgical devices of different types including, for example, the ultrasonic device **1004,** electrosurgical or RF surgical devices, such as, the RF device **1006,** and multifunction devices **1202** that integrate electrosurgical RF and ultrasonic energies delivered simultaneously from the generator **1002.** Although in the form of FIG. 1A, the generator **1002** is shown separate from the surgical devices **1004, 1006, 1202** in one form, the generator **1002** may be formed integrally with either of the surgical devices **1004, 1006, 1202** to form a unitary surgical system. The generator **1002** comprises an input device **1045** located on a front panel of the generator **1002** console. The input device **1045** may comprise any suitable device that generates signals suitable for programming the operation of the generator **1002.**

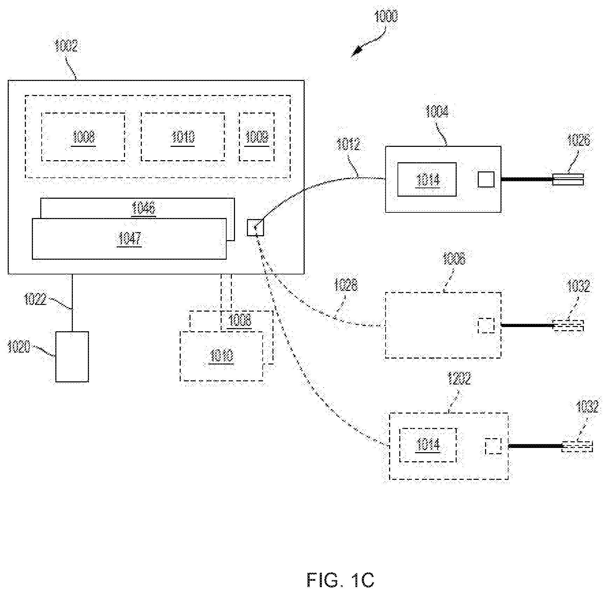

FIG. 1C is a diagram of the surgical system **10** of FIG. 1A. In various forms, the generator **1002** may comprise several separate functional elements, such as modules and/or blocks. Different functional elements or modules may be configured for driving the different kinds of surgical devices **1004, 1006, 1202.** For example, an ultrasonic generator module **1008** may drive ultrasonic devices such as the ultrasonic device **1004.** An electrosurgery/RF generator module **1010** may drive the electrosurgical device **1006.** For example, the respective modules **1008, 1010** may generate respective drive signals for driving the surgical devices **1004, 1006, 1202.** In various forms, the ultrasonic generator module **1008** and/or the electrosurgery/RF generator module **1010** each may be formed integrally with the generator **1002.** Alternatively, one or more of the modules **1008, 1010** may be provided as a separate circuit module electrically coupled to the generator **1002.** (The modules **1008** and **1010** are shown in phantom to illustrate this option.) Also, in some forms, the electrosurgery/RF generator module **1010** may be formed integrally with the ultrasonic generator module **1008,** or vice versa. Also, in some forms, the generator **1002** may be omitted entirely and the modules **1008, 1010** may be executed by processors or other hardware within the respective instruments **1004, 1006, 1202.**

In other forms, the electrical outputs of the ultrasonic generator module **1008** and the electrosurgery/RF generator module **1010** may be combined into a single electrical signal capable of driving the multifunction device **1202** simultaneously with electrosurgical RF and ultrasonic energies. The multifunction device **1202** comprises an ultrasonic transducer **1014** coupled to an ultrasonic blade and one or more electrodes in the end effector **1032** to receive electrosurgical RF energy. In such implementations, the combined RF/ultrasonic signal is coupled to the multifunction device **1202.** The multifunction device **1202** comprises signal processing components to split the combined RF/ultrasonic signal such that the RF signal can be delivered to the electrodes in the end effector **1032** and the ultrasonic signal can be delivered to the ultrasonic transducer **1014.**

In accordance with the described forms, the ultrasonic generator module **1008** may produce a drive signal or signals of particular voltages, currents, and frequencies, e.g., 55,500 cycles per second (Hz). The drive signal or signals may be provided to the ultrasonic device **1004,** and specifically to the transducer **1014,** which may operate, for example, as described above. The transducer **1014** and a waveguide extending through the shaft (waveguide not shown in FIG. 1B) may collectively form an ultrasonic drive system driving an ultrasonic blade **1017** of an end effector **1026.** In one form, the generator **1002** may be configured to produce a drive signal of a particular voltage, current, and/or frequency output signal that can be stepped or otherwise modified with high resolution, accuracy, and repeatability.

The generator **1002** may be activated to provide the drive signal to the transducer **1014** in any suitable manner. For example, the generator **1002** may comprise a foot switch **1020** coupled to the generator **1002** via a footswitch cable **1022.** A clinician may activate the transducer **1014** by depressing the foot switch **1020.** In addition, or instead of the foot switch **1020** some forms of the ultrasonic device **1004** may utilize one or more switches positioned on the hand piece that, when activated, may cause the generator **1002** to activate the transducer **1014.** In one form, for example, the one or more switches may comprise a pair of toggle buttons **1036a, 1036b** (Figure 1B), for example, to determine an operating mode of the device **1004.** When the toggle button **1036a** is depressed, for example, the ultrasonic generator **1002** may provide a maximum drive signal to the transducer **1014,** causing it to produce maximum ultrasonic energy output. Depressing toggle button **1036b** may cause the ultrasonic generator **1002** to provide a user-selectable drive signal to the transducer **1014,** causing it to produce less than the maximum ultrasonic energy output. The device **1004** additionally or alternatively may comprise a second switch (not shown) to, for example, indicate a position of a jaw closure trigger for operating jaws of the end effector **1026.** Also, in some forms, the ultrasonic generator **1002** may be activated based on the position of the jaw closure trigger, (e.g., as the clinician depresses the jaw closure trigger to close the jaws, ultrasonic energy may be applied).

Additionally or alternatively, the one or more switches may comprise a toggle button **1036c** that, when depressed, causes the generator **1002** to provide a pulsed output. The pulses may be provided at any suitable frequency and grouping, for example. In certain forms, the power level of the pulses may be the power levels associated with toggle buttons **1036a, 1036b** (maximum, less than maximum), for example.

It will be appreciated that a device **1004** may comprise any combination of the toggle buttons **1036a, 1036b, 1036c.** For example, the device **1004** could be configured to have only two toggle buttons: a toggle button **1036a** for producing maximum ultrasonic energy output and a toggle button **1036c** for producing a pulsed output at either the maximum or less than maximum power level. In this way, the drive signal output configuration of the generator **1002** could be 5 continuous signals and 5 or 4 or 3 or 2 or 1 pulsed signals. In certain forms, the specific drive signal configuration may be controlled based upon, for example, EEPROM settings in the generator **1002** and/or user power level selection(s).

In certain forms, a two-position switch may be provided as an alternative to a toggle button **1036c.** For example, a device **1004** may include a toggle button **1036a** for producing a continuous output at a maximum power level and a two-position toggle button **1036b.** In a first detented position, toggle button **1036b** may produce a continuous output at a less than maximum power level, and in a second detented position the toggle button **1036b** may produce a pulsed output (e.g., at either a maximum or less than maximum power level, depending upon the EEPROM settings).

In accordance with the described forms, the electrosurgery/RF generator module **1010** may generate a drive signal or signals with output power sufficient to perform bipolar electrosurgery using radio frequency (RF) energy. In bipolar electrosurgery applications, the drive signal may be provided, for example, to electrodes of the electrosurgical device **1006,** for example. Accordingly, the generator **1002** may be configured for therapeutic purposes by applying electrical energy to the tissue sufficient for treating the tissue (e.g., coagulation, cauterization, tissue welding).

The generator **1002** may comprise an input device **1045** (FIG. 1A) located, for example, on a front panel of the generator **1002** console. The input device **1045** may comprise any suitable device that generates signals suitable for programming the operation of the generator **1002.** In operation, the user can program or otherwise control operation of the generator **1002** using the input device **1045.** The input device **1045** may comprise any suitable device that generates signals that can be used by the generator (e.g., by one or more processors contained in the generator) to control the operation of the generator **1002** (e.g., operation of the ultrasonic generator module **1008** and/or electrosurgery/RF generator module **1010).** In various forms, the input device **1045** includes one or more of buttons, switches, thumbwheels, keyboard, keypad, touch screen monitor, pointing device, remote connection to a general purpose or dedicated computer. In other forms, the input device **1045** may comprise a suitable user interface, such as one or more user interface screens displayed on a touch screen monitor, for example. Accordingly, by way of the input device **1045,** the user can set or program various operating parameters of the generator, such as, for example, current (I), voltage (V), frequency (f), and/or period (T) of a drive signal or signals generated by the ultrasonic generator module **1008** and/or electrosurgery/RF generator module **1010.**

The generator **1002** may also comprise an output device **1047** (FIG. 1A), such as an output indicator, located, for example, on a front panel of the generator **1002** console. The output device **1047** includes one or more devices for providing a sensory feedback to a user. Such devices may comprise, for example, visual feedback devices (e.g., a visual feedback device may comprise incandescent lamps, light emitting diodes (LEDs), graphical user interface, display, analog indicator, digital indicator, bar graph display, digital alphanumeric display, LCD display screen, LED indicators), audio feedback devices (e.g., an audio feedback device may comprise speaker, buzzer, audible, computer generated tone, computerized speech, voice user interface (VUI) to interact with computers through a voice/speech platform), or tactile feedback devices (e.g., a tactile feedback device comprises any type of vibratory feedback, haptic actuator).

Although certain modules and/or blocks of the generator **1002** may be described by way of example, it can be appreciated that a greater or lesser number of modules and/or blocks may be used and still fall within the scope of the forms. Further, although various forms may be described in terms of modules and/or blocks to facilitate description, such modules and/or blocks may be implemented by one or more hardware components, e.g., processors, Digital Signal Processors (DSPs), Programmable Logic Devices (PLDs), Application Specific Integrated Circuits (ASICs), circuits, registers and/or software components, e.g., programs, subroutines, logic and/or combinations of hardware and software components. Also, in some forms, the various modules described herein may be implemented utilizing similar hardware positioned within the instruments **1004, 1006, 1202** (i.e., the generator **1002** may be omitted).

In one form, the ultrasonic generator drive module **1008** and electrosurgery/RF drive module **1010** may comprise one or more embedded applications implemented as firmware, software, hardware, or any combination thereof. The modules **1008, 1010** may comprise various executable modules such as software, programs, data, drivers, application program interfaces (APIs), and so forth. The firmware may be stored in nonvolatile memory (NVM), such as in bit-masked read-only memory (ROM) or flash memory. In various implementations, storing the firmware in ROM may preserve flash memory. The NVM may comprise other types of memory including, for example, programmable ROM (PROM), erasable programmable ROM (EPROM), electrically erasable programmable ROM (EEPROM), or battery backed random-access memory (RAM) such as dynamic RAM (DRAM), Double-Data-Rate DRAM (DDRAM), and/or synchronous DRAM (SDRAM).

In one form, the modules **1008, 1010** comprise a hardware component implemented as a processor for executing program instructions for monitoring various measurable characteristics of the devices **1004, 1006, 1202** and generating a corresponding output control signals for operating the devices **1004, 1006, 1202.** In forms in which the generator **1002** is used in conjunction with the device **1004,** the output control signal may drive the ultrasonic transducer **1014** in cutting and/or coagulation operating modes. Electrical characteristics of the device **1004** and/or tissue may be measured and used to control operational aspects of the generator **1002** and/or provided as feedback to the user. In forms in which the generator **1002** is used in conjunction with the device **1006,** the output control signal may supply electrical energy (e.g., RF energy) to the end effector **1032** in cutting, coagulation and/or desiccation modes. Electrical characteristics of the device **1006** and/or tissue may be measured and used to control operational aspects of the generator **1002** and/or provide feedback to the user. In various forms, as previously discussed, the hardware component may be implemented as a DSP, PLD, ASIC, circuits, and/or registers. In one form, the processor may be configured to store and execute computer software program instructions to generate the step function output signals for driving various components of the devices **1004, 1006, 1202,** such as the ultrasonic transducer **1014** and the end effectors **1026, 1032.**

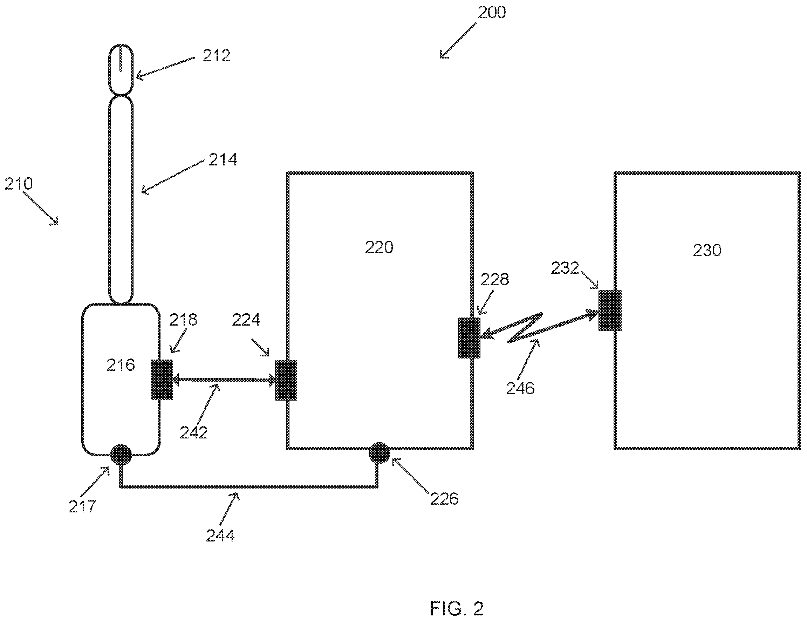

FIG. 2 illustrates a medical device system **200** that may include an electrosurgical medical device **210** and a medical device energy source **220.** The medical device **210** may include a hand-held component **216,** an end effector **212,** and an introducer or elongated shaft **214.** The medical device **210** may also include a device data interface **218** and a device power interface **217.** The medical device energy source **220** may also include complementary interfaces, including an energy source power interface **226** and an energy source data interface **224.**

The energy source power interface **226** may be configured to source electrical energy to the device power interface **217.** In some non-limiting examples, the electrical energy may be transmitted from the medical device energy source **220** to the medical device **210** by means of a power cable **244.** Further, the energy source data interface **224** may be configured to receive data from or transmit data to the device data interface **218.** Such data may be used by the medical device **210** to control one or more medical device functions. Alternatively, data from the medical device **210** may be transmitted from the device data interface **218** to the energy source data interface **224.** The data from the medical device **210** may be stored by the energy source **220** or may be used by the energy source **220** to control one or more energy source functions. In some non-limiting examples, the data transmitted from the medical device energy source **220** to the medical device **210,** or by the medical device **210** to the energy source **220,** may be accomplished by means of a data cable **242.**

In some non-limiting examples, the medical device data interface **218** and the energy source data interface **224** may include wireless interfaces. Such wireless interfaces may not require a data cable **242** for exchanging data between the energy source **220** and the medical device **210.**

In some non-limiting examples, the medical device data interface **218** and the medical device power interface **217** may be merged into a single medical device interface. Similarly, the energy source power interface **226** and the energy source data interface **224** may be merged into a single energy source interface. In such examples, a single power/data cable may be capable of transmitting both electrical energy and data.

The medical device system **200** may also include a medical device network server **230** having a network server communication interface **232.** The medical device network server **230** may store one or more databases of information related to the medical device **210,** the energy source **220,** and their respective functions. The medical device network server **230** may be in data communication with the energy source **220** via a network server communication interface **232** and an energy source network communication interface **228.** Data communication **246** between the medical device network server **230** and the energy source **220** may be accomplished through any standard data exchange method. Thus, the data communication **246** may include parallel communications methods, serial communications methods, optical communications methods, internet communications methods, wireless communication methods, and cellular communication methods. Although the medical device network server **230** is depicted in FIG. 2 as a single device, it may be recognized that the medical device network server **230** may comprise distributed servers, a cloud-based server, or other server configurations that may store the database.

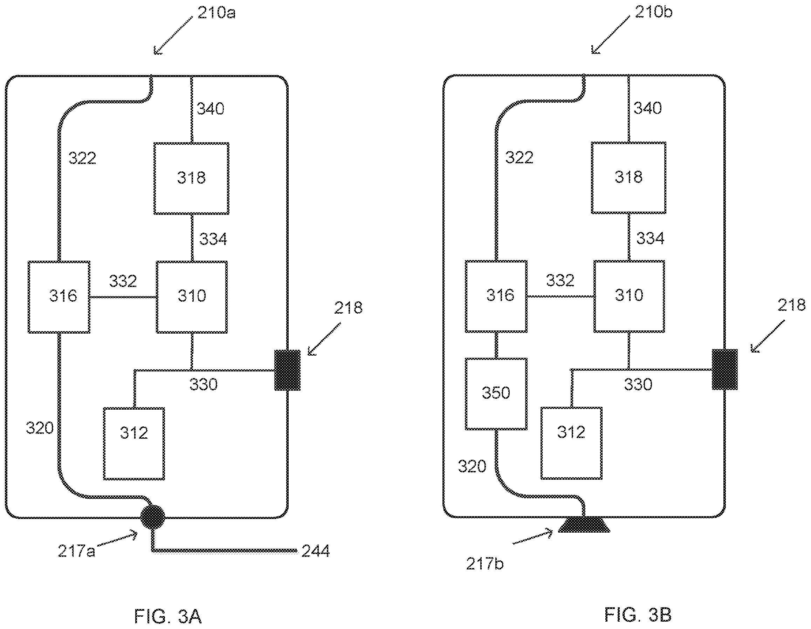

FIGS. 3A-B, 4A-B, and 5, disclosed below, depict exemplary components of the medical device system **200** greater detail. It may be understood that a medical device system **200** may not be limited to the specific components depicted in FIGS. 3A-B, 4A-B, and 5 and as disclosed below but may include additional components or lack certain components as disclosed.

FIGS. 3A and 3B depict two examples of a portion of a medical device, **210a** and **210b,** respectively. It may be recognized that the parts depicted in FIGS. 3A and 3B may be incorporated in the medical device hand-held component **216** or may be distributed throughout the medical device **210** as required.

FIG. 3A depicts a portion of a medical device **210a** having a device power interface **217a** configured to accept a power cable **244.** A device energy controller **316** may receive electrical energy from the device power interface **217a** through a device power bus **320.** The device energy controller **316** may be configured to regulate or control electrical power delivered to additional components of the medical device **210a** via a secondary device power bus **322.** In one non-limiting example, the device energy controller **316** may direct electrical energy to one or more electrodes at the medical device end effector **(1032** in FIG. 1C) for direct energy -- for example, RF energy -- application to a tissue. In another non-limiting example, the device energy controller **316** may direct electrical energy to one or more additional components, such as a piezoelectric component, configured to convert electrical energy to ultrasound energy. It may be recognized that the electrical energy received by the medical device **210a, 210b** may also be used to power a variety of electrical and/or electromechanical components found therein.

In general, the medical device **210a, 201b** may comprise various physical or logical elements implemented as hardware, software, or any combination thereof, as desired for a given set of design parameters or performance constraints. In various aspects, the physical or logical elements may be connected by one or more communications media. For example, communication media may comprise wired communication media (including one or more communication busses), wireless communication media, or a combination of both, as desired for a given implementation.

The medical device **210a, 201b** further comprises a device processor unit **310** and one or more device memory storage components **312.** The device processor unit **310** and the one or more memory storage components **312** may be in data communication via a device data bus **330.** The device data interface **218** may also be in data communication with the processor unit **310** and the one or more memory storage components **312** via the device data bus **330.** The device processor unit **310** may also be in communication with the device energy controller **316** over an energy control bus **332.** Alternatively, the device energy controller **316** may be in communication with the device processor unit **310** via the device data bus **330.**

The device data interface **218** may include any data communication interface that may be in data communication with the medical device energy source **220.** Such an interface may be a wired interface or a wireless interface. Wired communication modes include any mode of communication between points that utilizes wired technology including various protocols and combinations of protocols associated with wired transmission, data, and devices. Wireless communication modes include any mode of communication between points that utilizes, at least in part, wireless technology including various protocols and combinations of protocols associated with wireless transmission, data, and devices. Non-limiting examples of wired communication interfaces may include a serial interface, a parallel interface, an ethernet interface, and an optical cable interface. Non-limiting examples of a wireless interface may include a wireless local area network (WLAN) interface, a wireless wide area network (WWAN) interface, and a wireless personal area network (PAN) interface.

The device processor unit **310** may also control one or more electromechanical components **318** via one or more additional control lines **334.** The one or more electromechanical components **318** may include relays, motors, or other components configured to convert electrical energy into mechanical actuation. The mechanical actuation of the electromechanical components **318** may be transmitted via mechanical linkages **340** to other mechanical components of the medical device **210a, 210b** such as jaw actuators and cutting actuators at the end effector **(1032, 1026** in FIG. 1C).

The device processor unit **310** may also be in communication with one or more input and/or output interfaces of the medical device **210a, 201b.** Input interfaces may include, without limitation, push buttons, slide buttons, pressure sensors, heat sensors, magnetic sensors, light sensors, or other inputs associated with the medical device **210a, 201b** which may provide data regarding device use. Output interfaces may be used to activate, without limitation, LEDs, LED displays, LCD displays, audio indicators, haptic indicators, or other indicators to notify the user of the status of the medical device **210a, 201b.**

The device processor unit **310** may be implemented as a general purpose processor, a chip multiprocessor (CMP), a dedicated processor, an embedded processor, a digital signal processor (DSP), a microprocessor such as a complex instruction set computer (CISC) microprocessor, a reduced instruction set computing (RISC) microprocessor, and/or a very long instruction word (VLIW) microprocessor, or other processing device. The device processor unit **310** also may be implemented by a controller, a microcontroller, an application specific integrated circuit (ASIC), a field programmable gate array (FPGA), a programmable logic device (PLD), and so forth. In various aspects, the device processor unit **310** may be arranged to run an operating system (OS) and various mobile applications. Examples of an OS include, for example, operating systems generally known under the trade name of Microsoft Windows OS, and any other proprietary or open source OS.

In various aspects, the one or more device memory storage components **312** may comprise any machine-readable or computer-readable media capable of storing data, including both volatile and non-volatile memory. For example, memory may include read-only memory (ROM), random-access memory (RAM), dynamic RAM (DRAM), Double-Data-Rate DRAM (DDR-RAM), synchronous DRAM (SDRAM), static RAM (SRAM), programmable ROM (PROM), erasable programmable ROM (EPROM), electrically erasable programmable ROM (EEPROM), flash memory (e.g., NOR or NAND flash memory), content addressable memory (CAM), polymer memory (e.g., ferroelectric polymer memory), phase-change memory (e.g., ovonic memory), ferroelectric memory, silicon-oxide-nitride-oxide-silicon (SONOS) memory, or any other type of media suitable for storing information.

The device data bus **330** and the energy control bus **332** may be composed of any of several types of bus structure(s) including the memory bus or memory controller, a peripheral bus or external bus, and/or a local bus using any variety of available bus architectures including, but not limited to, 9-bit bus, Industrial Standard Architecture (ISA), Micro-Charmel Architecture (MSA), Extended ISA (EISA), Intelligent Drive Electronics (IDE), VESA Local Bus (VLB), Peripheral Component Interconnect (PCI), Universal Serial Bus (USB), Advanced Graphics Port (AGP), Personal Computer Memory Card International Association bus (PCMCIA), Small Computer Systems Interface (SCSI) or other proprietary bus.

The one or more device memory storage components **312** may be used to store instructions that may be executed by the device processor unit 310. The instructions may include any suitable type of code, such as source code, compiled code, interpreted code, executable code, static code, dynamic code, and the like. The instruction or a set of instructions may include those that, if executed by the device processor unit **310,** may cause the device processor unit **310** to perform a method and/or operations in accordance with the disclosure. The instructions may be implemented using any suitable high-level, low-level, object-oriented, visual, compiled and/or interpreted programming language, such as C, C++, Java, BASIC, Perl, Matlab, Pascal, Visual BASIC, assembly language, machine code, and so forth.

Instructions stored in the one or more device memory storage components **312** may include instructions to control an amount of energy delivered by the device energy controller **316** to the end effector **212.** Other instructions may control operations of the one or more electromechanical components **318.** Additional instructions may cause the device processor unit **310** to store information associated with the use of the medical device **210a, 201b.** Use information may include, without limitation, a number of times energy is delivered by the end effector **212** to a single piece of tissue or to multiple pieces of tissue, data related to an amount of energy delivered to a tissue by the end effector **212** for each application of energy to the tissue, and a length of time during with the amount of energy is delivered to the tissue. Additional use information may be stored, including, without limitation, temperature of a tissue receiving energy and an impedance measurement of the tissue.

The one or more device memory storage components **312** may also store data associated with the medical device **210a, 201b** and its use. As disclosed above, data associated with device use may include, without limitation, a number of times energy is delivered by the end effector **212** to a single piece of tissue or to multiple pieces of tissue, data related to an amount of energy delivered to a tissue by the end effector **212** for each application of energy to the tissue, and a length of time during with the amount of energy is delivered to the tissue. Additional use information may be stored, including, without limitation, temperature of a tissue receiving energy and an impedance measurement of the tissue.

The one or more device memory storage components **312** may also store information characterizing the medical device **210a, 201b.** Such characterizing information may include, without limitation, a device name, a device model number, a device lot or serial number, a device use limitation number, a device power limitation number, a device expiration date, and a device identifier code. The device identifier code may include one or more machine readable characters, or one or more strings of such machine readable characters. The device identifier code may be composed of a single string of machine readable characters. Alternatively, the device identifier code may be compose of a plurality of strings of machine readable characters, such as, as non-limiting examples, two strings of machine readable characters or three strings of machine readable characters. In one non-limiting example, the device identifier code may comprise a string of machine readable characters related to the device characterizing information. In another non-limiting example, the device identifier code may comprise one or more strings of randomly generated machine readable characters. The device identifier code may be composed of one or more strings of any number of machine readable characters. Non-limiting examples of the number of machine readable characters in each of the one or more strings of the device identifier code may include 32 characters, 64 characters, 128 characters, 256 characters, 512 characters, or any number of characters therebetween including endpoints.

FIG. 3B depicts a portion of a medical device **210b** having a device power interface **217b** configured to interface with a medical device energy source through an energy docking port. The medical device **210b** having a docking power interface **217b** may be operated free of a power cable **244** for easier handling. It may be observed that many of the components of medical device **210b** are identical to those of medical device **210a** as disclosed above. A cordless medical device **210b** may include a rechargeable battery **350** to receive and store power received from a medical device energy source **220** while the medical device **210b** is physically docked to the medical device energy source **220.** Power from the battery **350** may be regulated through the device energy controller **316** in a manner suitable for power use for such a cordless medical device **210b.** Data communication between the cordless medical device **210b** and a medical device energy source **220** may be accomplished through a device data interface **218** which may be composed of a wired communication interface or a wireless communication interface as disclosed above with respect to a corded medical device **210a.**

It should be understood that a corded medical device **210a** that accepts electrical energy via a power cable **244** may also include a battery for electrical power storage. Such additional power storage capability may be used as a separate power source for electrical and/or electromechanical components of the medical device **210a.** Such a battery may be used to electrically isolate the electrical or electronic components from noise on the device power bus **320** during the operation of the device. Alternatively, such a battery may serve as a back-up power supply to the electronic components in the event of a power failure of the medical device energy source **220.**

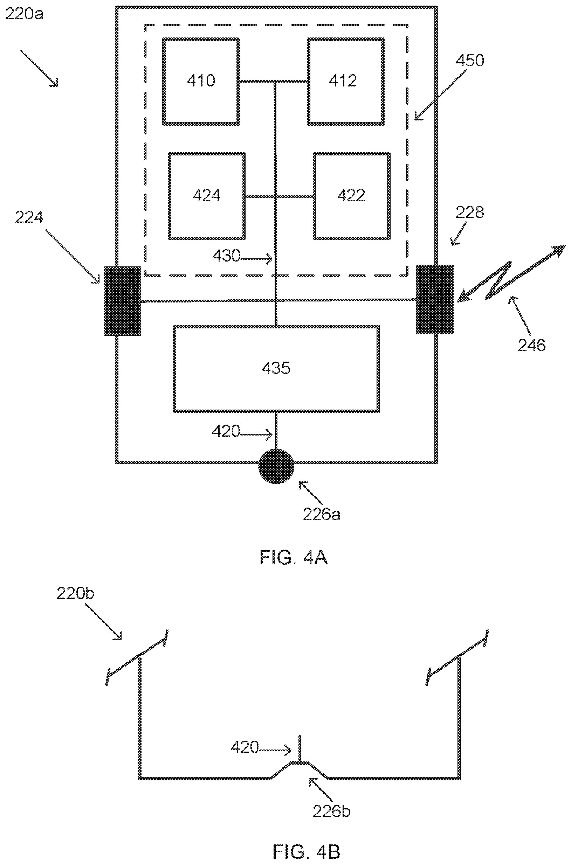

FIGS. 4A and 4B depict two examples of a medical device energy source, **220a** and **220b,** respectively. FIG. 4A depicts an example of a medical device energy source **220a** that may be used with a corded medical device **210a.** FIG. 4B depicts a portion of an exemplary medical device energy source **220b** that may be used with a cordless medical device **210b.**

As depicted in FIG. 4A, a medical device energy source **220a** may incorporate an energy source **435** in electrical communication via an energy source device power bus **420** to an energy source power interface **226a** configured to deliver electrical energy from the energy source. Energy source power interface **226a** may be suitable for an attached power cable **244** configured to conduct electrical energy to a corded medical device **210a.** The energy source **435** may be controlled to supply an effective amount of electrical energy to the medical device **210.** An effective amount of electrical energy may comprise a therapeutic amount of energy, a non-therapeutic amount of energy, or both a therapeutic and a non-therapeutic amount of energy to the medical device. Non-limiting examples of a therapeutic amount of energy may include an amount of energy required to effect a therapy on a tissue, such as an amount of energy to cauterize a tissue, an amount of energy to shrink a tissue, or an amount of energy to cut a tissue according to the type of medical device **210** receiving the electrical energy. Non-limiting examples of a non-therapeutic amount of energy may include an amount of energy that is not sufficient to effect a therapy on a tissue including an amount of energy to measure a tissue impedance or an amount of energy to power electronic components of the medical device **210a.** The electrical energy sourced by the energy source **435** may be controlled with respect to a DC voltage, an AC voltage, an RMS voltage, a DC current, an AC current, an RMS current, a frequency, a pulse-width modulation, or any combination thereof.

The energy source **435** may be controlled by an energy source computing device **450.** The energy source computing device **450** may be composed of an energy source processor unit **410,** one or more energy source memory storage components **412,** one or more energy source input interfaces **424,** one or more energy source output interfaces **422,** and an energy source data bus **430** It may be understood that the energy source data bus **430** may be configured to place the one or more of the energy source memory storage components **412,** one or more energy source input interfaces **424,** and one or more energy source output interfaces **422** in operative communication with the energy source processor unit **410.**

The one or more energy source memory storage components **412** may be used to store instructions that may be executed by the energy source processor unit **410.** Some non-limiting examples of such instructions may include: instructions to receive data from the one or more energy source input interfaces **424;** instructions related to a display of information on display devices that may be in operative communication with the energy source output interfaces **422;** instructions to control the operation of the energy source **435;** instructions to transmit data to the medical device via the energy source data interface **224;** instructions to receive data from the medical device via the energy source data interface **224;** instructions to determine that the medical device **210** is in functional communication with the medical device energy source **220;** instructions that the energy source power interface **226a,b** in in operative communication with the device power interface **217a,b;** and instructions to determine that the energy source data interface **224** is in operative communication with the device data interface **218.** Additional instructions may cause the energy source **435** not to deliver an effective amount of electrical energy via the energy source power interface **226a,b** to the medical device **210a,b** when the medical device is not in functional communication with the medical device energy source. The instructions may include any suitable type of code, such as source code, compiled code, interpreted code, executable code, static code, dynamic code, and the like. The instruction or a set of instructions may include those that, if executed by the energy source processor unit **410,** may cause the energy source processor unit 410 to perform a method and/or operations in accordance with the disclosure. The instructions may be implemented using any suitable high-level, low-level, object-oriented, visual, compiled and/or interpreted programming language, such as C, C++, Java, BASIC, Perl, Matlab, Pascal, Visual BASIC, assembly language, machine code, and so forth.

The energy source input interfaces **424** may comprise any interface configured to provide input from a user to the energy source computing device **450** via energy source data bus **430.** Non-limiting examples of such an input interface **424** may include a serial interface, a parallel interface, an audio (microphone) interface, a wireless interface including an RF interface, and an optical interface. Such an energy source input interface **424** may be in data communication with any number of user actuators including, without limitation, push buttons, slide buttons, keyboards, knobs, touch screens, and computer mice. A user may employ the actuators to direct the operation of the medical device energy source **220a,** for example setting a maximum amount of electrical energy to be supplied by the energy source **435,** or a number of times the energy source **435** may supply electrical energy to the medical device **210a.**

The energy source output interface **422** may comprise any interface configured to provide information to a user to the energy source computing device **450** via energy source data bus **430.** The user display may thus be in operative communication with the energy source processor unit **410** by means of the energy source output interface **422** and the energy source bus **430.** Non-limiting examples of such an output interface **422** may include a serial interface, a parallel interface, a video interface, an audio (speaker) interface, a wireless interface including an RF interface, and an optical interface. Such energy source output interfaces **422** may be in data communication with any number of display or communication devices including, without limitation, LEDs, LED displays, LCD displays, plasma displays, audio annunciators, and speakers. The display or communication devices may be configured to provide information to a user regarding the use of the medical device energy source **220a** including an amount of electrical energy sourced by the energy source **435** during a medical procedure, an indication of a fault condition of the energy source **435,** or an indication that the medical device **210** is not an appropriate device to receive electrical energy from the energy source **435.**

The energy source data bus **430** may be configured to transfer data and/or information among the components of the energy source computing device **450.** The energy source data bus **430** may be configured to direct instructions to the energy source **435** to cause the energy source **435** to regulate the amount of electrical energy sourced therefrom. The energy source data bus **430** may also be configured to transmit data to and receive data from the energy source data interface **224,** thereby permitting exchange of data with the medical device **210a.** The energy source data interface **224** may thus be in operative communication with the energy source processor unit **410** by means of the energy source data bus **430.** The energy source data bus **430** may further be configured to transmit data to and receive data from the energy source network communication interface **228,** thereby permitting exchange of data with a medical device network server **230.** The energy source network communication interface **228** may thus be in operative communication with the energy source processor unit **410** by means of the energy source data bus **430.**

It may be recognized that the energy source processor unit **410** may include similar devices as those disclosed above with respect to the medical device processor unit **310.** Additionally, the one or more energy source memory storage components **412** may include similar devices as those disclosed above with respect to the medical device memory storage components **312.** Further, the energy source data bus **430** may include similar devices as those disclosed above with respect to the device data bus **330.** It may also be recognized that the energy source data interface **224** may include complementary components to those disclosed above with respect to the device data interface **218.** In some non-limiting examples, the energy source data interface **224** may comprise one or more of a serial data interface, a parallel data interface, a wireless interface, and an optical interface

The energy source network communication interface **228** may comprise any interface configured to permit information exchange **246** with one or more networked server devices, such as medical device network server **230.** The medical device network server **230** may be logically connected to the medical device energy source **220a** through the energy source network communication interface **228.** The energy source network communication interface **228** may encompass any known interface including, without limitation, a wired internet interface, a wireless internet interface, a WiFi interface, a BlueTooth interface, a LAN interface, a WAN interface, a telephonic interface, a cellular interface, and an optical interface. The communication interface may permit communication among networks such as local-area networks (LAN) and wide area networks (WAN). Non-limiting examples of LAN technologies may include Fiber Distributed Data Interface (FDDI), Copper Distributed Data Interface (CDDI), Ethernet/IEEE 802.3, Token Ring/IEEE 802.5 and the like. Non-limiting examples of WAN technologies may include, but are not limited to, point-to-point links, circuit switching networks like Integrated Services Digital Networks (ISDN) and variations thereon, packet switching networks, and Digital Subscriber Lines (DSL).

The energy source network communication interface **228** may further comprise wireless or cellular communications interfaces. Examples of wireless protocols may include various wireless local area network (WLAN) protocols, including the Institute of Electrical and Electronics Engineers (IEEE) 802.xx series of protocols, such as IEEE 802.11a/b/g/n, IEEE 802.16, IEEE 802.20, and so forth. Other examples of wireless protocols may include various wireless wide area network (WWAN) protocols, such as GSM cellular radiotelephone system protocols with GPRS, CDMA cellular radiotelephone communication systems with 1xRTT, EDGE systems, EV-DO systems, EV-DV systems, HSDPA systems, and so forth. Further examples of wireless protocols may include wireless personal area network (PAN) protocols, such as an Infrared protocol, a protocol from the Bluetooth Special Interest Group (SIG) series of protocols, including Bluetooth Specification versions v1.0, v1.1, v1.2, v2.0, v2.0 with Enhanced Data Rate (EDR), as well as one or more Bluetooth Profiles, and so forth. Yet another example of wireless protocols may include near-field communication techniques and protocols, such as electro-magnetic induction (EMI) techniques. An example of EMI techniques may include passive or active radio-frequency identification (RFID) protocols and devices. Other suitable protocols may include Ultra Wide Band (UWB), Digital Office (DO), Digital Home, Trusted Platform Module (TPM), ZigBee, and so forth.

Examples of cellular communication systems may include CDMA cellular radiotelephone communication systems, GSM cellular radiotelephone systems, North American Digital Cellular (NADC) cellular radiotelephone systems, Time Division Multiple Access (TDMA) cellular radiotelephone systems, Extended-TDMA (E-TDMA) cellular radiotelephone systems, Narrowband Advanced Mobile Phone Service (NAMPS) cellular radiotelephone systems, third generation (3G) systems such as WCDMA, CDMA-2000, UMTS cellular radiotelephone systems compliant with the Third-Generation Partnership Project (3GPP), and so forth.

As disclosed above, FIG. 4A depicts a medical device energy source **220a** having an energy source power interface **226a** suitable for conducting electrical energy to a corded medical device **210a** via an attached power cable **244.** FIG. 4B depicts a portion of a medical device energy source **220b** having an energy source power interface **226b** configured to provide electrical energy to a cordless medical device **210b** which may store the power in a battery **350.** The medical device energy source **220b** may include an energy source power interface **226b** such as a docking station that may be complementary to the device power interface **217b** of the cordless medical device **210b.** The energy source power interface **226b** may also receive electrical energy from energy source **435** via energy source device power bus **420.** It may be understood that the components and operations disclosed above with respect to medical device energy source **220a** and its components as depicted in FIG. 4A may also be similar to those in the medical device energy source **220b** as depicted in FIG. 4B (with the exception of the energy source power interface **226a).** In one non-limiting example, medical device energy source **220b** may be configured to exchange data with the medical device **210b** through the energy source data interface **224** over a data cable **242** in communication with the medical device data interface **218.** In another non-limiting example, the energy source data interface **224** of medical device energy source **220b** may communicate data with the medical device data interface **218** over a wireless interface. It may be recognized that wireless communication between medical device energy source **220b** and medical device **210b** may result in medical device **210b** having no physical attachments to medical device energy source **220b,** permitting unencumbered used of medical device **210b.**

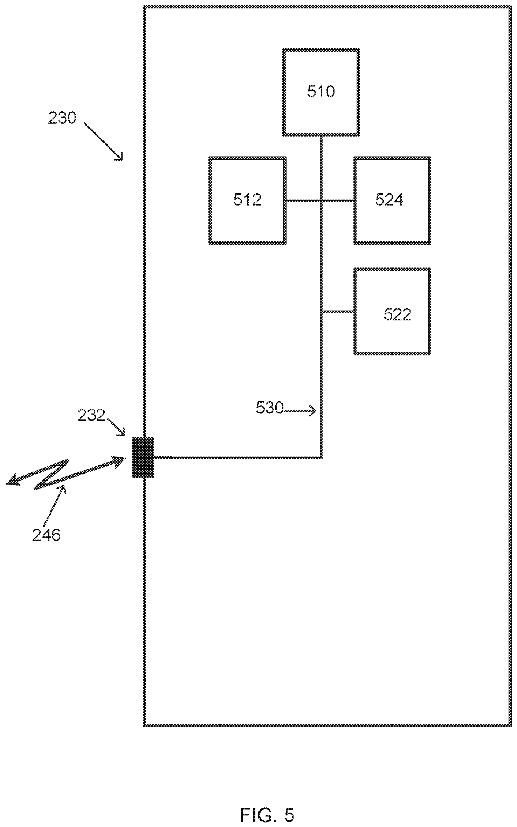

FIG. 5 depicts a block diagram of an example of a medical device network server **230.** The medical device network server **230** may be composed of a medical device network server processor unit **510,** one or more medical device network server memory storage components **512,** one or more medical device network server input interfaces **524,** one or more medical device network server output interfaces **522,** and a medical device network server data bus **530.** The medical device network server **230** may have a network server communication interface **232.** The medical device network server processor unit **510** may be in operative communication with the one or more medical device network server memory storage components **512** and the network server communication interface **232** via the network server data bus **530.** The medical device network server **230** may store one or more databases of information related to the medical device **210a,b,** the energy source **220a,b,** and their respective functions. The database may be stored in the one or more medical device network server memory storage components **512.** The one or more medical device network server memory storage components **512** may also include instructions that may cause the medical device network sever processor unit **510** to operate according to those instructions. In some non-limiting examples, medical device network server memory storage components **512** may include instructions: to receive a request for database data from a medical device energy source; to transmit to the medical device energy source all or a portion of data from the database; to update the status indicator of a medical device in the database according to the medical device identity code; and to update usage data of a medical device in the database according to the medical device identity code. The medical device network server **230** may be in data communication with the energy source **220a,b** via a network server communication interface **232** and an energy source network communication interface **228.** Data communication **246** between the medical device network server **230** and the energy source **220a,b** may be accomplished through any standard data exchange method. The network server communication interface **232** may comprise interfaces and protocols complementary to those disclosed above with respect to the energy source network communication interface **228.**

It may be recognized that the medical device network server processor unit **510** may include similar devices as those disclosed above with respect to the medical device processor unit **310.** Additionally, the one or more medical device network server memory storage components **512** may include similar devices as those disclosed above with respect to the medical device memory storage components **312.** Further, the medical device network server data bus **530** may include similar devices as those disclosed above with respect to the device data bus **330.** It may also be recognized that the medical device network server input interfaces **524** and one or more medical device network server output interfaces **522** may include similar devices and data exchange protocols as those disclose above with respect to the energy source input interface **424** and energy source output interface **422,** respectively.

The one or more medical device network server memory storage components **512** may be used to store instructions that may be executed by the medical device network server processor unit **510.** The instructions may include any suitable type of code, such as source code, compiled code, interpreted code, executable code, static code, dynamic code, and the like. The instruction or a set of instructions may include those that, if executed by the medical device network server processor unit **510**, may cause the medical device network server processor unit **510** to perform a method and/or operations in accordance with the disclosure. The instructions may be implemented using any suitable high-level, low-level, object-oriented, visual, compiled and/or interpreted programming language, such as C, C++, Java, BASIC, Perl, Matlab, Pascal, Visual BASIC, assembly language, machine code, and so forth.

A medical device network server input interface **524** may be in data communication with any number of user actuators including, without limitation, push buttons, slide buttons, keyboards, knobs, touch screens, and computer mice. A user may employ the actuators to direct the operation of the medical device network server **230**, for example to input or modify a database that may be stored in the medical device network server memory storage components **512.**

The medical device network server output interface **522** may comprise an interface configured to provide information to a user of the medical device network server **230** via medical device network server data bus **530.** Non-limiting examples of such an output interface **522** may include a serial interface, a parallel interface, a video interface, an audio (speaker) interface, a wireless interface including an RF interface, and an optical interface. Such medical device network server output interfaces **522** may be in data communication with any number of display or communication devices including, without limitation, LEDs, LED displays, LCD displays, plasma displays, audio annunciators, and speakers. The display or communication devices may be configured to provide information to a user regarding the status of the medical device network server **230** or information relevant to the database stored in the medical device network server memory storage components **512.**

As disclosed above, it may be recognized that proper control of the electrical energy supplied to an electrosurgical device may be critical for safe and effective operation of the device. It is therefore desirable for a medical device energy source to supply an appropriate amount of electrical energy to an electrosurgical device to promote a safe and effective therapeutic outcome. An example of a medical device system **(200,** FIG. 2) that may be used towards this end is one in which a medical device energy source **(220,** FIG. 2) may obtain information from a medical device **(210,** FIG. 2) and compare that information with data maintained in a database stored in a medical device network server **(230,** FIG. 2). As a result of that comparison, the medical device energy source may determine, based on software instructions stored in the energy source memory storage component **(412,** FIG. 4A), an amount of electrical energy to supply to the medical device. The medical device energy source may receive from the medical device an identifier code via the energy source data interface **(224,** FIG. 2). The medical device energy source may also receive **(246,** FIG. 2) data from the medical device network server via an energy source network communication interface **(228,** FIG. 2). These data may include data from a database stored in the medical device network server memory storage component **512.**

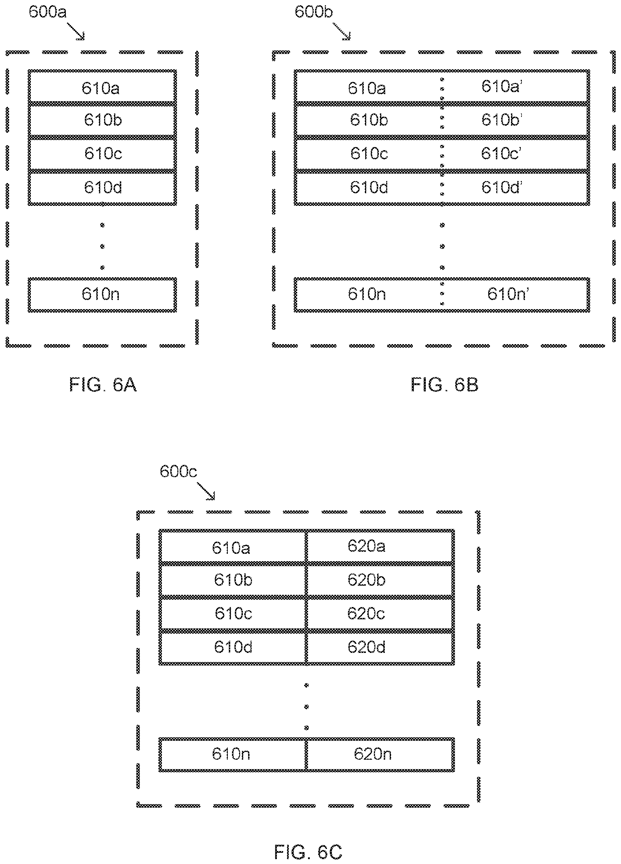

FIGS. 6A-6D depict exemplary structures of the database. It may be recognized that the examples depicted in FIGS. 6A-6D are non-limiting, and that the database may have any appropriate structure for maintaining and organizing the data.

FIG. 6A depicts a database **600a** comprising a list of identity codes **610a-610n,** in which each identity code is associated with a separate medical device. The number of identity codes **610n** that may be stored in the database **600a** may include any finite number of identity codes. For example, the number of identity codes may range from 1 identity code to about 100 identity codes. Non-limiting examples of the number of identity codes that may be including in the database 600a may include 1 identity code, 2 identity codes, 5 identity codes, 10 identity codes, 20 identity codes, 50 identity codes, 100 identity codes, or ranges in values therebetween including endpoints. As depicted in FIG. 6A, these identity codes **601a-610n** may each comprise a single string of processor readable characters. Each string may include any number of processor readable characters, which may range from about 8 processor readable characters to about 256 processor readable characters. Non-limiting examples of the number of processor readable characters may include 8 processor readable characters, 16 processor readable characters, 32 processor readable characters, 64 processor readable characters, 128 processor readable characters, 256 processor readable characters, or ranges in number of processor readable characters therebetween including endpoints. Although the number of processor readable characters disclosed above are powers of 2 (that is, 2ⁿ, where n has an integer value of 3 to 8), it may be recognized that the number of processor readable characters that comprise an identity code may include any finite integer number of processor readable characters. In one non-limiting example, each string of processor readable characters that comprises an identity code **601a-610n** may comprise a random or pseudo-random string of processor readable characters. In another non-limiting example, each string of processor readable characters that comprises an identity code **601a-610n** may comprise a string of processor readable characters that encode data related to one of several medical devices. Examples of data that may be suitable for such encoding may include, without limitation, a medical device name, a medical device model number, a medical device serial number, a medical device date of manufacture, a medical device expiration date, or combination or combinations thereof.

FIG. 6B depicts a database **600b** comprising a list of identity codes **610a-610n** in which each identity code is associated with a separate medical device. The number of such identity codes **610a-610n** in database **600b** may be similar to the number of identity codes as disclosed above with respect to database **600a** (FIG. 6A). As depicted in FIG. 6B, these identity codes **610a-610n** may each comprise two individual strings (generically denoted as **610x** and **610x')** of processor readable characters. Thus, each identity code may comprise a first string **610a, 610b ... 610n** and a second string **610a', 610b'...610n',** respectively. It may be understood that although FIG. 6B depicts a database **600b** composed of identity codes each having two separate strings of processor readable characters, the number of strings of processor readable characters for each identity code is not limiting, and may include 2 strings, 3 strings, 4 strings, or any finite number of strings of processor readable characters. Each string of processor readable characters that comprises an identity code in database **600b** may be characterized in a similar manner as disclosed above with respect to the identity codes in database **600a** (FIG. 6A). It may further be recognized the each of the two individual strings (generically denoted as **610x** and **610x')** comprising the identity codes may have the same number of processor readable characters or a different number of processor readable characters. Similarly, an identifier code stored in the medical device memory component may comprise multiple strings each string having the same number of processor readable characters or a different number of processor readable characters.

FIG. 6C depicts a database **600c** comprising a set of two data fields, each of which may be associated with a separate medical device. Thus, for example, a first medical device may be associated with data fields **610a** and **620a,** a second medical device may be associated with data fields **610b** and **620b,** and similar for a medical device associated with data fields **610n** and **620n.** The number of such paired data fields **(610a,620a)** through **(610n,620n)** in database **600c** may be similar to the number of identity codes as disclosed above with respect to database **600a** (FIG. 6A). In some non-limiting examples, the first data field, comprising data **610a, 610b,** ... **610n,** may comprise an identity code associated with a medical device characterized as above with respect to FIGS. 6A (database **600a)** and 6B (database **600b).**

The second data field, comprising data **620a, 620b,** ... **620n,** may be associated with a second characterizer of the medical device with which the database entry is associated. In one non-limiting example, the second data field may include an indicator of a medical device status. The indicator may include a text descriptor of the status or one or more processor readable characters that may encode the status. Examples of such status indicators may include, without limitation, "NEW," "UNUSED," "USED," or "REFURBISHED." Such status indicators may be used to identify the device as being new (for example, new out of the box), an unused device (previously attached to the energy source, but not used in any medical procedure), a device used in a medical procedure, and a device that had been used but was then refurbished (for example sterilized, cleaned, mechanically adjusted) for potential reuse. It may be understood that additional or alternative status indicators may also be included. Alternatively, a second data field may include alternative characterizations of the medical device including, without limitation, a device product number, a device serial number, a device lot number, an expiration date, or any other characterization of the medical device. Although database **600c** is depicted as comprising two sets of data fields associated with each medical device, it may be recognized that the database **600c** may include any number of sets of data fields associated with each medical device, including 3 sets, 4 sets, or any finite number of sets of data fields.

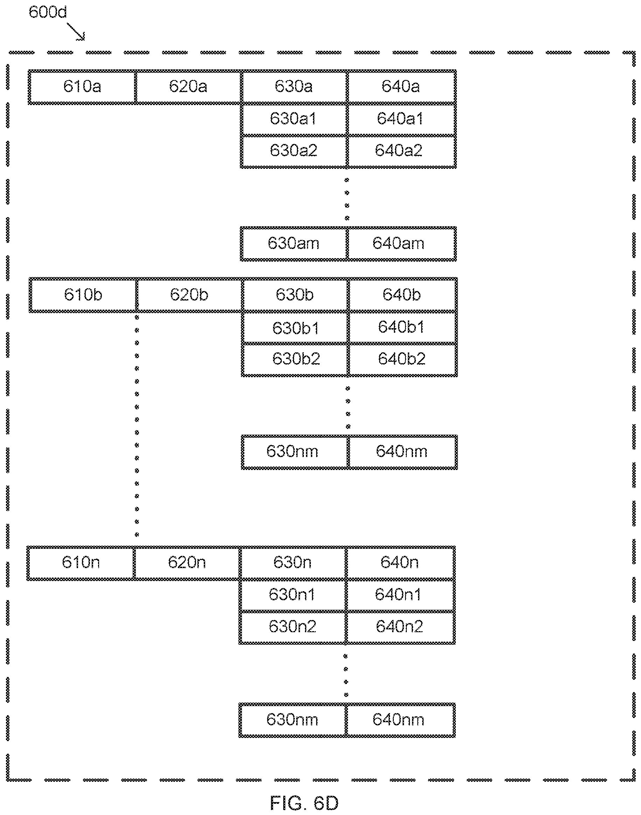

FIG. 6D depicts a more complex database **600d.** Database **600d** may comprise multiple data fields **(610a-610n, 620a-620n, 630a-630n,** and **640a-640n),** in which an entry in each data field is associated with a specific medical device. Some of the fields (for example data field **610** and **620)** may comprise a single data entry for each associated medical device, for example a medical device identity code **(610a)** and a medical device status indicator **(620a).** Other data fields **(630a-n** and **640a-n)** may include multiple data entries for each medical device stored in sub-fields. As depicted in FIG. 6D, data field **630a** may include sub-fields such as **630a1, 630a2,** ...., **630am** and data field **640a** may include sub-fields such as **640a1, 640a2,** ...., **630am,** wherein m has a finite integer value and represents the number of sub-field entries.

Data fields comprising sub-fields may be useful to retain historical data related to the use of a medical device. For example, a data field may include a total number of allowed uses of a device **(630a),** such as 5 total uses, and each subfield may include an individual use number, such as the number 1 in sub-field **630a1,** the number 2 in sub-field **630a2,** and so forth. Other types of data may include a total power permitted to be sourced to a medical device (for example, in data field **640a)** as well as the amount of power sourced to the device for each use, for a first use in data field **640a1,** for a second use in data field **640a2,** and so forth. Other exemplary information that may be stored in such data fields comprising sub-fields may include a total time of medical device use, a maximum amount of time that the medical device may be used, and an amount of time for each use. It may be recognized that the examples of data stored in the data fields and sub-fields are not limiting, but may include any data related to the use of a given medical device.

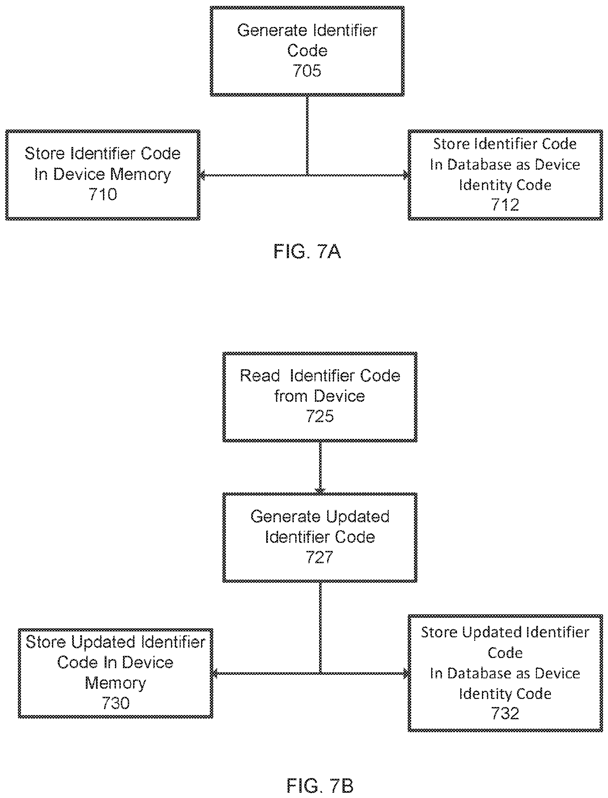

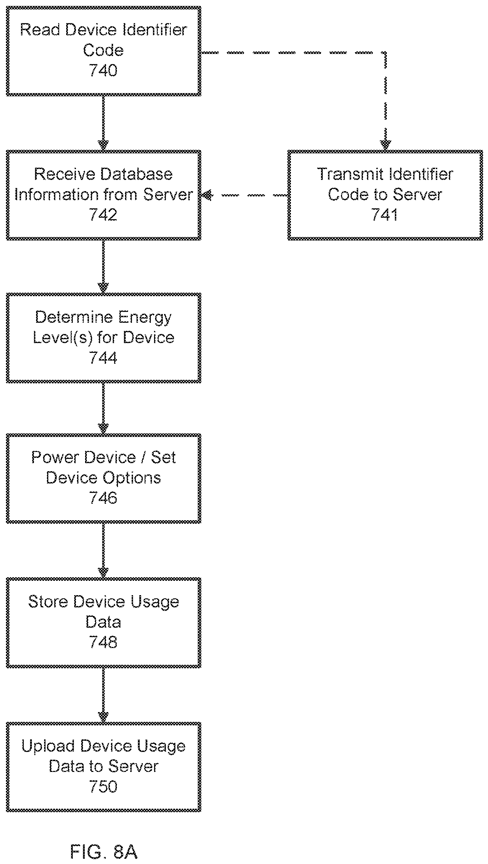

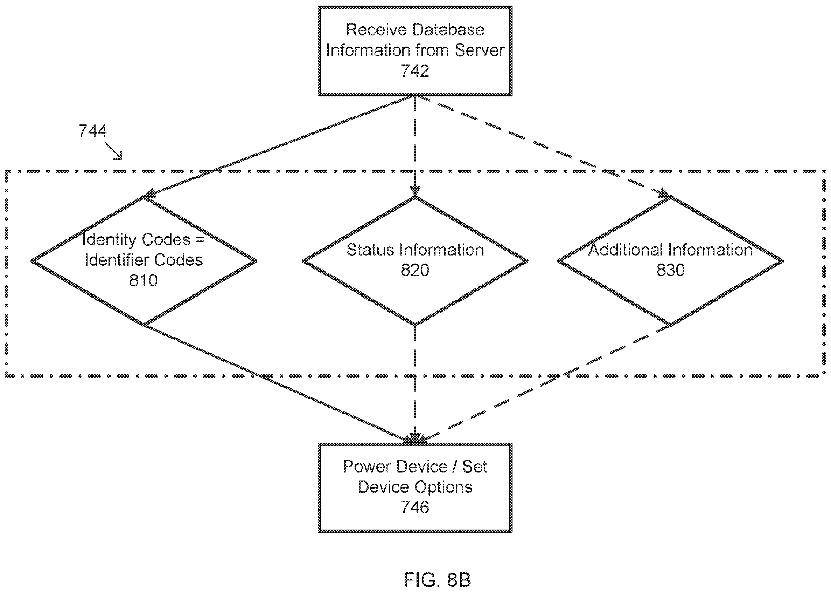

FIGS. 7A, 7B, 8A, and 8B are flow diagrams of methods related to the use of the medical device system disclosed herein. In one non-limiting example, these methods (not claimed as such) consider a medical device fabricated at a manufacturer's facility (or a facility of a third party approved by the manufacturer) which may be sold or leased to an end user such as a medical professional or a health care facility. The device may be a single use device or a multi-use (re-usable) device. After each medical use, a multi-use device may require maintenance that may include, without limitation, physical cleaning, sterilization, functional recertification (for example that moving parts operate correctly, or that electrical contacts to tissue meet required electrical specifications), or combinations thereof. The maintenance may be carried out at a facility owned and/or operated by the manufacturer or a facility owned and/or operated by a third party that is approved and certified by the manufacturer.

FIG. 7A is a flow diagram of one non-limiting example of a method (not claimed as such) related to an initial manufacture of a medical device. As part of the manufacturing process, a unique identifier code may be generated **705** for each medical device. As disclosed above, the identifier code may comprise one or more strings of processor readable characters of a defined length, and may comprise random characters or characters that encode characterizing information about the medical device. The identifier code may then be stored in the device memory storage component **710.** Additionally, the identifier code may be programmed into the database stored in the medical device network server, for example as a new database entry, as a medical device identity code **712.**

Depending on the database structure (see, for example, FIGS. 6A-6D), additional information may be added to the database as part of the device manufacturing process. For example, the database may include a status field, which may be programmed with an appropriate entry (such as "NEW"). Data associated with device history such as the maximum number of allowed uses, maximum energy to be supplied by the device, and maximum time for the device to be actively used may also be entered into the appropriate data fields and sub-fields. Further, additional fields in the database that may characterize the device -- model number, lot number, serial number, date of manufacture, and expiration date -- may be populated with data appropriate to the newly manufactured device.

It may be noted that the medical device network server may be under the sole control of the manufacturer. In one non-limiting example, the database stored in the medical device network server may be accessible to only a limited number of employees of the manufacturer. In another non-limiting example, employees of a certified or licensed third party (for example, a third party contracted to refurbish or recertify a medical device) may also have access to the database. It may be understood that software instructions stored in the network server memory storage component may be used to limit or control access to the database by the manufacturer or third party employees according to protocols known in the art.

FIG. 7B is a flow diagram of one non-limiting example of a method (not claimed as such) related to a maintenance procedure for a multi-use medical device. In one non-limiting example, the identifier code of a particular medical device may be retained after the maintenance procedure has been completed. In such an example, the equivalent identity code in the database may be retained. In an alternative non-limiting example, the identifier code of a device may be read **725** from the device memory storage component and a new or updated identifier code may be generated **727.** The new or updated identifier code may be stored in the device memory storage component **730.** Similarly, the new or updated identifier code may be stored in the database as a new or updated identity code **732.** In one non-limiting example, the new identity code may replace the previous identity code in the database. In an alternative non-limiting example, the new identity code may be added as a new entry in the database, and the previous identity code may be retained or removed.

It may be understood, that additional data in the database may be updated, changed, or deleted as part of the maintenance procedure. For example, a status indicator associate with the medical device in the database may be set to indicate that the device has been refurbished or re-certified. Data that may be associated with the historical use of the device prior to the maintenance procedure (such as the prior number of actual uses, amount of time associated with the use of the device, and power supplied by the device) may be deleted from the database. Alternatively, the prior historical use data may be retained. Additional data related to the maintenance procedure may also be added to the database in one or more maintenance fields. Non-limiting examples of maintenance related data may include a date of maintenance, the number of times a maintenance procedure has been performed on the medical device, the name of the facility performing the maintenance, the name(s) of personnel recertifying the device, testing data associated with device recertification, or combinations thereof.

FIG. 8A is a flow diagram of one non-limiting example of a method (not claimed as such) related to the use of a medical device system by a health care professional during a medical procedure.

A medical device may be contacted with a medical device energy source. Such contact may include affixing data cables and power cables between the two devices. Alternatively, such contact may include docking a cordless medical device with the medical device energy source, and causing a wireless data connection to be made between the two devices. The medical device energy source may be powered before the medical device is contacted with the medical device energy source, or may be powered after the medical device is contacted with the medical device energy source. Additionally, the medical device energy source, on being powered, may establish a communication link with a medical device network server over an appropriate communication channel (including one or more communication interfaces and one or more communication protocols).

After the medical device is contacted with the medical device energy source, the medical device energy source may read the device identifier code **740** from the medical device. In some non-limiting examples, the medical device may receive the device identifier code **740** via an energy source data interface. The device identifier code may be stored in a device memory storage component and may be received by the medical device energy source via an energy source data interface in operative communication with device data interface.

In one non-limiting alternative example, the medical device energy source may transmit the identifier code to the medical device network server **741.** The medical device network server may compare the identifier code with one or more identity codes stored in the database. The medical device network server may respond by transmitting database information to the medical
device energy source that is associated with a medical device having an identity code equal to the identifier code.

Alternatively, the medical device energy source may transmit a request to the medical device network server to receive data associated with the database. The medical device network server may respond to the request by transmitting all or a portion of the database information stored in the memory storage component of the medical device network server to the medical device energy source.

In either example, the medical device energy source may receive the database information from the server **742.** The medical device energy source may include instructions that, when executed by the medical device processor, causes the medical device energy source to determine the energy level(s) to supply to the device **744** which may be based, in part, on a comparison of the device identifier code with a device identity code supplied by the medical device network server from the database.

The medical device energy source may set an appropriate power level for delivery to the medical device and/or set device options **746** of the medical device. The power level may be set by the energy source based on control instructions received from the medical device energy source computing device. As disclosed above, the energy level may comprise a therapeutic or non-therapeutic level of power. Non-limiting examples of a therapeutic amount of energy may include an amount of energy required to effect a therapy on a tissue, such as an amount of energy to cauterize a tissue, an amount of energy to shrink a tissue, or an amount of energy to cut a tissue according to the type of medical device receiving the electrical energy. Non-limiting examples of a non-therapeutic amount of energy may include an amount of energy that is not sufficient to effect a therapy on a tissue including an amount of energy to measure a tissue impedance or an amount of energy to power electronic components of the medical device. The electrical energy sourced by the energy source may be controlled with respect to a DC voltage, an AC voltage, an RMS voltage, a DC current, an AC current, an RMS current, a frequency, a pulse-width modulation, or any combination thereof.

While the medical device is being used, the medical device energy source may store some amount of medical device usage data **748** in the energy source memory storage component. Non-limiting examples of such usage data may include: a total number of times the medical device is energized with an amount of energy, the amount of energy supplied to the medical device for each energization step, the total amount of energy supplied to the medical device, the length of time the energy is supplied to the medical device for each energization step, and measurement data collected by the medical device before, during, or after each energization step. Non-limiting examples of such measurement data may include a tissue impedance value and a tissue temperature value.

The usage data obtained by the medical device energy source may be uploaded to the medical device network server **750.** Such data may be uploaded during the medical procedure in which the medical device is being used or after the use of the medical device. Additional information may be uploaded to the medical device network server including, without limitation, a time stamp, a date stamp, a facility identifier (identifying the facility in which the medical device is used), and/or any other additional information related to the identity of the medical device being used, the circumstances under which the device is used, and the location in which the device is used. Additionally, the medical device energy source may upload data to the medical device network server to update the database for indicators including, but not limited to, the device status. In some examples, the medical device energy source may upload data to the medical device network server to update the device status to indicate that the device is unused or used.

It may be understood that one important feature of the a method (not claimed as such) related to the use of a medical device system as disclosed above is the step of determining the energy level(s) supplied by the medical device energy source to the medical device 744. FIG.8B is a flow chart that suggests some of the functions of the medical device energy source that may be used to make this determination. Although three specific examples of such determinations are depicted in FIG. 8B, it may be recognized that additional or alternative determinations may be made by the medical device energy source depending on a variety of information and data obtained from the medical device, the medical device network server, and/or a medical device energy source user through one or more of the energy source input devices (via the one or more energy source input interfaces).

As depicted in FIG. 8B the medical device energy source may receive database information from the medical device network server **742** via an energy source network communication interface. The medical device energy source may determine the energy level(s) to supply to the medical device **744** based at least in part on the database information. As depicted in FIGS. 6A-6D, the database may comprise one of a variety of structures depending on the type and amount of information stored therein. The determination of the energy level supplied to the medical device **744** may be based on the type of data presented by the medical device network server as determined by the database structure.

In one non-limiting example, the medical device energy source may receive data having a database structure depicted in FIG. 6A and 6B from the medical device network server via an energy source network communication interface. The medical device energy source may receive from the medical device network server a list of identity codes that may comprise one (for example **610a-610n** in FIG. 6A) or more (for example **610a, 610a'-610n, 610n'** in FIG. 6B) strings of processor readable characters as disclosed above. The medical device energy source may then compare the list of identity codes to the identifier code received by the medical device energy source from the medical device. The medical device energy source may then determine the energy level to supply to the medical device based on determining if any one of the identity codes is equal to the identifier code **810.** It may be understood that, in the case in which the identity code and the identifier code each comprise multiple strings of processor readable characters, the medical device energy source will compare each of the multiple strings comprising the identity code and identifier code. In such an example, the medical device energy source may only supply a therapeutic amount of energy to the medical device if the medical device identifier code is equal to one of the identity codes listed in the database. When the medical device energy source determines that the identifier code is the same as at least one of the identity codes, the medical device energy source may deliver a therapeutic amount of energy, a non-therapeutic amount of energy, or a combination of therapeutic and non-therapeutic amount of energy to the medical device. Alternatively, when the medical device energy source determines that the identifier code is the not same as at least one of the identity codes, the medical device energy source may deliver no energy or only a non-therapeutic amount of energy to the medical device.

In another non-limiting example, the medical device energy source may receive data having a database structure depicted in FIG. 6C from the medical device network server. Such a database may include device status information **620a-620n** in FIG. 6C along with the identity codes **610a-610n** in FIG. 6C. The medical device energy source may determine if any one of the identity codes equal the identifier codes **810** as disclosed above. In addition, the medical device energy source may determine an amount of energy level(s) to source to the medical device **744** based on the status information **820** corresponding to a medical device identity code that is equal to the medical device identifier code, in addition to the equality of the identifier and identity codes **810.** For example, the identifier code of a medical device may be the same as an identity code included in the data base, but the status of that medical device may indicate that it has been used, and therefore should not be reused. As a result, although the medical device may be a listed device in the database, the medical device energy source may include instructions not to supply a therapeutic amount of energy to the medical device because it is used and has not been refurbished. Alternatively, the identifier code of a medical device may be the same as an identity code included in the data base, and the status of that medical device may indicate that it is new, unused or refurbished. The medical device energy source may include instructions to permit an effective or therapeutic amount of energy to be supplied to devices having such status indicators.

In yet another non-limiting example, the medical device energy source may receive data from a database structure depicted in FIG. 6D. Such a database may include additional device data maintained in a plurality of database fields such as **630 (630a1-630am** through **630n1-630nm)** and **640 (640a-640am** through **640n-640nm)** along with device status information **620a-620n** and the identity codes **610a-610n.** The medical device energy source may determine if any one of the identity codes equal the identifier codes **810** as disclosed above. The medical device energy source may also consider the status information **820** associated with the medical device. Further, the medical device energy source may determine the amount of energy to source to the medical device based on the additional data **830.**

As one example, the medical device identifier code may be listed among the acceptable medical device identity codes, and the medical device may have a status of "NEW" or "UNUSED." However, if an attempt is made to use the medical device after an expiration date (as determined from one of the additional fields in the database), the medical device energy source may not supply a therapeutic amount of energy to the medical device. In another example, a medical device energy source may receive additional data as part of a database from the medical device network server concerning the number of times a medical device may used or the amount of energy that may be sourced to the medical device for each use of the device or a total amount of energy that may be sourced to the medical device. During the use of the medical device, the medical device energy source may track the number of uses of the device, the amount of energy supplied during each use, and the amount of time during which the device is energized. Once a use limit has been reached -- for example, the number of times the device is energized, an amount of time during which the device is energized, or the total amount of energy sourced to the device -- the medical device energy source may be programmed to cease sourcing additional therapeutic energy to the device.

It will be appreciated that the terms "proximal" and "distal" are used throughout the specification with reference to a clinician manipulating one end of an instrument used to treat a patient. The term "proximal" refers to the portion of the instrument closest to the clinician and the term "distal" refers to the portion located furthest from the clinician. It will further be appreciated that for conciseness and clarity, spatial terms such as "vertical," "horizontal," "up," or "down" may be used herein with respect to the illustrated embodiments. However, surgical instruments may be used in many orientations and positions, and these terms are not intended to be limiting or absolute.

Various aspects of surgical instruments and robotic surgical systems are described herein. It will be understood by those skilled in the art that the various aspects described herein may be used with the described surgical instruments and robotic surgical systems. The descriptions are provided for example only, and those skilled in the art will understand that the disclosed examples are not limited to only the devices disclosed herein, but may be used with any compatible surgical instrument or robotic surgical system.

Reference throughout the specification to "various aspects," "some aspects," "one example," or "one aspect" means that a particular feature, structure, or characteristic described in connection with the aspect is included in at least one example. Thus, appearances of the phrases "in various aspects," "in some aspects," "in one example," or "in one aspect" in places throughout the specification are not necessarily all referring to the same aspect. Furthermore, the particular features, structures, or characteristics illustrated or described in connection with one example may be combined, in whole or in part, with features, structures, or characteristics of one or more other aspects without limitation.

While various aspects herein have been illustrated by description of several aspects and while the illustrative embodiments have been described in considerable detail, it is not the intention of the applicant to restrict or in any way limit the scope of the appended claims to such detail. Additional advantages and modifications may readily appear to those skilled in the art. For example, it is generally accepted that endoscopic procedures are more common than laparoscopic procedures. Accordingly, the present invention has been discussed in terms of endoscopic procedures and apparatus. However, use herein of terms such as "endoscopic", should not be construed to limit the present invention to an instrument for use only in conjunction with an endoscopic tube (e.g., trocar). On the contrary, it is believed that the present invention may find use in any procedure where access is limited to a small incision, including but not limited to laparoscopic procedures, as well as open procedures.

It is to be understood that at least some of the figures and descriptions herein have been simplified to illustrate elements that are relevant for a clear understanding of the disclosure, while eliminating, for purposes of clarity, other elements. Those of ordinary skill in the art will recognize, however, that these and other elements may be desirable. However, because such elements are well known in the art, and because they do not facilitate a better understanding of the disclosure, a discussion of such elements is not provided herein.

## Claims

1. A medical device energy source (220b), comprising:
an energy source (435);
an energy source power interface (226b) configured to deliver electrical energy from the energy source (435); and
an energy source computing device (450), comprising:
an energy source processor unit (410);
an energy source memory storage component (412) in operative communication with the energy source processor unit;
an energy source network communication interface (228) in operative communication with the energy source processor unit; and
an energy source data interface (224) in operative communication with the energy source processor unit,
wherein the energy source computing device (450) is configured to control a function of the energy source, and
wherein, the energy source memory storage component (412) comprises instructions that, when executed by the energy source processor unit, cause the energy source computing device to:
receive an identifier code via the energy source data interface;
receive a plurality of medical device identity codes via the energy source network communication interface;
compare the identifier code with each of the plurality of medical device identity codes; and
control the function of the energy source based on the comparison of the identifier code with each of the plurality of medical device identity codes.

2. The medical device energy source of claim 1, wherein the identifier code comprises two identifier strings (610x, 610x'), each of the two identifier strings comprising a string of processor readable characters.

3. The medical device energy source of claim 2, wherein the instructions that cause the energy source computing device to compare the identifier code with each of the plurality of medical device identity codes comprises instructions that cause the energy source computing device to compare each of the two identifier strings with each of two identity strings comprising each of the medical device identity codes.

4. The medical device energy source of claim 1, wherein, the energy source memory storage component comprises instructions that, when executed by the energy source processor unit, further cause the energy source computing device to receive, via the energy source network communication interface, a plurality of medical device status indicators, wherein each medical device status indicator corresponding to each of the plurality of medical device identity codes,
wherein optionally the instructions that cause the energy source computing device to control the function of the energy source further comprise instructions that cause the energy source computing device to control the function of the energy source based on the medical device status indicators corresponding to a medical device identity code equal to the identifier code.

5. The medical device energy source of claim 1, wherein the energy source memory storage component comprises instructions that, when executed by the energy source processor unit, further cause the energy source computing device to retain, in the energy source memory storage component:
an energizer value corresponding to an amount of energy supplied by the energy source;
an energizer time value corresponding to a length of time during which the energy source supplies an amount of energy;
an energizer number corresponding to a number of times the energy source supplies an amount of energy;
or combinations thereof.

6. The medical device energy source of claim 1, further comprising a user display in operative communication with the energy source processor unit.

7. A medical device system, comprising:
a medical device (210b), comprising:
a device memory storage component (312) configured to store an identifier code;
a device data interface (218) in operative connection with the memory storage component; and
a device power interface (217b) configured to receive electric power from an energy source (435);
a medical device energy source (220b), comprising:
the energy source (435);
an energy source power interface (226b) in operative communication with the device power interface and configured to deliver electrical energy from the energy source to the medical device; and
an energy source computing device (450), comprising:
an energy source processor unit (410);
an energy source memory storage component (412) in operative communication with the energy source processor unit;
an energy source network communication interface (228) in operative communication with the energy source processor unit and configured to transmit data to and receive data from a communication network; and
an energy source data interface (224) in operative connection with the device data interface,
wherein the energy source computing device is configured to control a function of the energy source; and
a medical device network server (230), comprising:
a network server processor unit (510);
a network server memory storage component (512) in operative communication with the network server processor unit and configured to store a medical device database comprising a plurality of medical device identity codes and corresponding medical device status indicators; and
a network server communication interface (232) in operative communication with the network server processor unit and configured to transmit data to and receive data from at least one medical device power source via the communication network;
wherein, the energy source memory storage component comprises instructions that, when executed by the energy source processor unit, cause the energy source computing device to:
receive, from the device memory storage component, the identifier code;
receive, from the network server memory storage component, the plurality of medical device identity codes from the medical device database;
compare the identifier code with each of the plurality of medical device identity codes; and
control the function of the energy source based on the comparison of the at least one identifier code with the plurality of medical device identity codes.

8. The medical device system of claim 7, wherein the identifier code comprises two identifier strings, each of the two identifier strings (610x, 610x') comprising a string of processor readable characters, and
wherein the instructions that cause the energy source computing device to compare the identifier code with each of the plurality of medical device identity codes comprises instructions that cause the energy source computing device to compare each of the two identifier strings with each of two identity strings comprising each of the medical device identity codes.

9. The medical device system of claim 7, wherein, the energy source memory storage component comprises instructions that, when executed by the energy source processor unit, further cause the energy source computing device to receive, via the energy source network communication interface, a plurality of medical device status indicators, wherein each of the plurality of medical device status indicators corresponds to each of the plurality of medical device identity codes, and
wherein the instructions that cause the energy source computing device to control the function of the energy source further comprise instructions that cause the energy source computing device to control the function of the energy source based on a medical device status indicator corresponding to a medical device identity code equal to the identifier code.

10. The medical device system of claim 7, wherein the energy source memory storage component further comprises instructions that, when executed by the energy source processor unit, cause the energy source computing device to transmit, to the medical device network server, data to update a medical device status indicator corresponding to a medical device identity code equal to the identifier code.

11. The medical device system of claim 10, wherein the network server memory storage component comprises instructions that, when executed by the network servicer processor unit, cause the network server processor unit to:
receive, from the medical device energy source, data to update a medical device status indicator corresponding to the medical device identity code equal to the identifier code; and
update the status indicator in the data base corresponding to the medical device identity code equal to the identifier code.

12. The medical device system of claim 7, wherein the medical device database further comprises one or more additional indicators corresponding to each of the medical device identity codes in the medical device database.

13. The medical device system of claim 7, wherein the energy source memory storage component further comprises instructions that, when executed by the energy source processor unit, cause the energy source computing device to store in the energy source memory storage component:
an indicator of total medical device uses;
an indicator, for each use of the total medical device uses, of:
an amount of power supplied by the medical device energy source to the medical device; and
a length of time during which the medical device energy source supplies the amount of energy to the medical device;
a total amount of power supplied by the medical device energy source to the medical device over the total medical device uses;
or any combination thereof.

14. The medical device system of claim 13, wherein the energy source memory storage component further comprises instructions that, when executed by the energy source processor unit, cause the energy source computing device to:
receive, from the medical device network server, values of the one or more additional indicators corresponding to each of the medical device identity codes in the medical device data base; and
control the function of the energy source based on the value of the one or more of the additional indicators corresponding to the medical device identity code equal to the identifier code.

15. The medical device system of claim 13, wherein the network server memory storage component comprises instructions that, when executed by the network servicer processor unit, cause the network server processor unit to:
receive, from the medical device energy source, values of the one or more additional indicators corresponding to each of the medical device identity codes in the medical device data base; and
update the values of the one or more additional indicators corresponding to each of the medical device identity codes in the medical device data base.

## Patentansprüche

1. Energiequelle (220b) einer medizinischen Vorrichtung, umfassend:
eine Energiequelle (435);
eine Energiequellenstromschnittstelle (226b), die konfiguriert ist, um elektrische Energie von der Energiequelle (435) zu liefern; und
eine Energiequellenrechenvorrichtung (450), umfassend:
eine Energiequellenprozessoreinheit (410);
eine Energiequellenspeicherkomponente (412), die in operativer Kommunikation mit der Energiequellenprozessoreinheit steht;
eine Energiequellennetzwerkkommunikationsschnittstelle (228), die in operativer Kommunikation mit der Energiequellenprozessoreinheit steht; und
eine Energiequellendatenschnittstelle (224), die in operativer Kommunikation mit der Energiequellenprozessoreinheit steht,
wobei die Energiequellenrechenvorrichtung (450) konfiguriert ist, um eine Funktion der Energiequelle zu steuern, und
wobei die Energiequellenspeicherkomponente (412) Anweisungen umfasst, die, wenn sie durch die Energiequellenprozessoreinheit ausgeführt werden, die Energiequellenrechenvorrichtung veranlassen zum:
Empfangen eines Identifikationscodes über die Energiequellendatenschnittstelle;
Empfangen einer Vielzahl von Identitätscodes der medizinischen Vorrichtung über die Energiequellennetzwerkkommunikationsschnittstelle;
Vergleichen des Identifikationscodes mit jedem der Vielzahl von Identitätscodes der medizinischen Vorrichtung; und
Steuern der Funktion der Energiequelle basierend auf dem Vergleich des Identifikationscodes mit jedem der Vielzahl von Identitätscodes der medizinischen Vorrichtung.

2. Energiequelle der medizinischen Vorrichtung nach Anspruch 1, wobei der Identifikationscode zwei Identifikationszeichenfolgen (610x, 610x') umfasst, jede der zwei Identifikationszeichenfolgen umfassend eine Zeichenfolge prozessorlesbarer Zeichen.

3. Energiequelle der medizinischen Vorrichtung nach Anspruch 2, wobei die Anweisungen, die die Energiequellenrechenvorrichtung veranlassen, den Identifikationscode mit jedem der Vielzahl von Identitätscodes der medizinischen Vorrichtung zu vergleichen, Anweisungen umfassen, die die Energiequellenrechenvorrichtung veranlassen, jede der zwei Identifikationszeichenfolgen mit jeder der zwei Identitätszeichenfolgen zu vergleichen, umfassend jeden der Identitätscodes der medizinischen Vorrichtung.

4. Energiequelle der medizinischen Vorrichtung nach Anspruch 1, wobei die Energiequellenspeicherkomponente Anweisungen umfasst, die, wenn sie durch die Energiequellenprozessoreinheit ausgeführt werden, die Energiequellenrechenvorrichtung ferner veranlassen, über die Energiequellennetzwerkkommunikationsschnittstelle, eine Vielzahl von Statusindikatoren der medizinischen Vorrichtung zu empfangen, wobei jeder Statusindikator der medizinischen Vorrichtung jedem der Vielzahl von Identitätscodes der medizinische Vorrichtung entspricht,
wobei optional die Anweisungen, die die Energiequellenrechenvorrichtung veranlassen, die Funktion der Energiequelle zu steuern, ferner Anweisungen umfassen, die die Energiequellenrechenvorrichtung veranlassen, die Funktion der Energiequelle basierend auf den Statusindikatoren der medizinischen Vorrichtung zu steuern, die einem Identitätscode der medizinischen Vorrichtung entsprechen, der gleich dem Identifikationscode ist.

5. Energiequelle der medizinischen Vorrichtung nach Anspruch 1, wobei die Energiequellenspeicherkomponente Anweisungen umfasst, die, wenn sie durch die Energiequellenprozessoreinheit ausgeführt werden, ferner die Energiequellenrechenvorrichtung, in der Energiequellenspeicherkomponente, veranlassen, beizubehalten:
einen Energieverstärkerwert, der einer Energiemenge entspricht, die durch die Energiequelle geliefert wird;
einen Energieverstärkerzeitwert, der einer Zeitspanne entspricht, während der die Energiequelle eine Energiemenge liefert;
eine Energieverstärkeranzahl, die der Anzahl von Malen entspricht, die die Energiequelle eine Energiemenge liefert;
oder Kombinationen davon.

6. Energiequelle der medizinischen Vorrichtung nach Anspruch 1, ferner umfassend eine Benutzeranzeige, die in operativer Kommunikation mit der Prozessoreinheit der Energiequelle steht.

7. System der medizinischen Vorrichtung, umfassend:
eine medizinische Vorrichtung (210b), umfassend:
eine Vorrichtungsspeicherkomponente (312), die konfiguriert ist, um einen Identifikationscode zu speichern;
eine Vorrichtungsdatenschnittstelle (218), die in operativer Verbindung mit der Speicherkomponente steht; und
eine Vorrichtungsstromschnittstelle (217b), die konfiguriert ist, um elektrischen Strom von einer Energiequelle (435) zu empfangen;
eine Energiequelle (220b) der medizinischen Vorrichtung, umfassend:
die Energiequelle (435);
eine Energiequellenstromschnittstelle (226b), die in operativer Kommunikation mit der Vorrichtungsstromschnittstelle steht und konfiguriert ist, um elektrische Energie von der Energiequelle an die medizinische Vorrichtung zu liefern; und
eine Energiequellenrechenvorrichtung (450), umfassend:
eine Energiequellenprozessoreinheit (410);
eine Energiequellenspeicherkomponente (412), die in operativer Kommunikation mit der Energiequellenprozessoreinheit steht;
eine Energiequellennetzwerkkommunikationsschnittstelle (228), die in operativer Kommunikation mit der Energiequellenprozessoreinheit steht und konfiguriert ist, um Daten an ein Kommunikationsnetzwerk zu übertragen und Daten von einem Kommunikationsnetzwerk zu empfangen; und
eine Energiequellendatenschnittstelle (224), die in operativer Verbindung mit der Vorrichtungsdatenschnittstelle steht,
wobei die Energiequellenrechenvorrichtung konfiguriert ist, um eine Funktion der Energiequelle zu steuern; und
einen Netzwerkserver (230) der medizinischen Vorrichtung, umfassend:
eine Netzwerkserverprozessoreinheit (510);
eine Netzwerkserverspeicherkomponente (512), die in operativer Kommunikation mit der Netzwerkserverprozessoreinheit steht und konfiguriert ist, um eine Datenbank der medizinischen Vorrichtung, umfassend eine Vielzahl von Identitätscodes der medizinischen Vorrichtung und entsprechende Indikatoren der medizinischen Vorrichtung umfasst; und
eine Netzwerkserverkommunikationsschnittstelle (232), die in operativer Kommunikation mit der Netzwerkserverprozessoreinheit steht und konfiguriert ist, um Daten zu mindestens einer Stromquelle der medizinischen Vorrichtung über das Kommunikationsnetzwerk zu übertragen und Daten von dieser zu empfangen; wobei die Energiequellenspeicherkomponente Anweisungen umfasst, die, wenn sie durch die Energiequellenprozessoreinheit ausgeführt werden, die Energiequellenrechenvorrichtung veranlassen zum:
Empfangen, von der Vorrichtungsspeicherkomponente, des Identifikationscodes;
Empfangen, von der Netzwerkserverspeicherkomponente, der Vielzahl von Identitätscodes der medizinischen Vorrichtung aus der Datenbank der medizinischen Vorrichtung;
Vergleichen des Identifikationscodes mit jedem der Vielzahl von Identitätscodes der medizinischen Vorrichtung; und
Steuern der Funktion der Energiequelle basierend auf dem Vergleich des mindestens einen Identifikationscodes mit der Vielzahl von Identifikationscodes der medizinischen Vorrichtung.

8. System der medizinischen Vorrichtung nach Anspruch 7, wobei der Identifikationscode zwei Identifikationszeichenfolgen umfasst, jede der zwei Identifikationszeichenfolgen (610x, 610x') umfassend eine Zeichenfolge von prozessorlesbaren Zeichen, und
wobei die Anweisungen, die die Energiequellenrechenvorrichtung veranlassen, den Identifikationscode mit jedem der Vielzahl von Identitätscodes der medizinischen Vorrichtung zu vergleichen, Anweisungen umfassen, die die Energiequellenrechenvorrichtung veranlassen, jede der zwei Identifikationszeichenfolgen mit jeder der zwei Identitätszeichenfolgen zu vergleichen, umfassend jeden der Identitätscodes der medizinischen Vorrichtung.

9. System der medizinischen Vorrichtung nach Anspruch 7, wobei die Energiequellenspeicherkomponente Anweisungen umfasst, die, wenn sie durch die Energiequellenprozessoreinheit ausgeführt werden, das Energiequellenrechenvorrichtung ferner veranlassen, über die Energiequellennetzwerkkommunikationsschnittstelle eine Vielzahl von Statusindikatoren der medizinischen Vorrichtung zu empfangen, wobei jeder der Vielzahl von Statusindikatoren der medizinischen Vorrichtung jedem der Vielzahl von Identitätscodes der medizinischen Vorrichtung entspricht, und
wobei die Anweisungen, die die Energiequellenrechenvorrichtung veranlassen, die Funktion der Energiequelle zu steuern, ferner Anweisungen umfassen, die die Energiequellenrechenvorrichtung veranlassen, die Funktion der Energiequelle basierend auf einem Statusindikator der medizinischen Vorrichtung zu steuern, der einem Identitätscode der medizinischen Vorrichtung entspricht, der gleich dem Identifikationscode ist.

10. System der medizinischen Vorrichtung nach Anspruch 7, wobei die Energiequellenspeicherkomponente ferner Anweisungen umfasst, die, wenn sie durch die Energiequellenprozessoreinheit ausgeführt werden, die Energiequellenrechenvorrichtung veranlassen, an den Netzwerkserver der medizinischen Vorrichtung, Daten zu übertragen, um einen Statusindikator der medizinischen Vorrichtung zu aktualisieren, der einem Identitätscode der medizinischen Vorrichtung entspricht, der gleich dem Identifikationscode ist.

11. System der medizinischen Vorrichtung nach Anspruch 10, wobei die Netzwerkserverspeicherkomponente Anweisungen umfasst, die, wenn sie durch die Netzwerkserverprozessoreinheit ausgeführt werden, die Netzwerkserverprozessoreinheit veranlassen zum:
Empfangen, von der Energiequelle der medizinischen Vorrichtung, von Daten, um einen Statusindikator der medizinischen Vorrichtung zu aktualisieren, der dem Identitätscode der medizinischen Vorrichtung entspricht, der gleich dem Identifikationscode ist; und
Aktualisieren des Statusindikators in der Datenbank, der dem Identitätscode der medizinischen Vorrichtung entspricht, der gleich dem Identifikationscode ist.

12. System der medizinischen Vorrichtung nach Anspruch 7, wobei die Datenbank der medizinischen Vorrichtung ferner einen oder mehrere zusätzliche Indikatoren umfasst, die jedem der Identitätscodes der medizinischen Vorrichtung in der Datenbank der medizinischen Vorrichtung entsprechen.

13. System der medizinischen Vorrichtung nach Anspruch 7, wobei die Energiequellenspeicherkomponente ferner Anweisungen umfasst, die, wenn sie durch die Energiequellenprozessoreinheit ausgeführt werden, die Energiequellenrechenvorrichtung veranlassen, in der Energiequellenspeicherkomponente zu speichern:
ein Indikator einer Gesamtverwendung der medizinischen Vorrichtung;
einen Indikator, für jede Verwendung der Gesamtverwendung der medizinischen Vorrichtung, von:
einer Strommenge, die durch die Energiequelle der medizinischen Vorrichtung an die medizinische Vorrichtung geliefert wird; und
eine Zeitspanne, während der die Energiequelle der medizinischen Vorrichtung die Energiemenge an die medizinische Vorrichtung liefert;
eine Gesamtmenge an Strom, die durch die Energiequelle der medizinischen Vorrichtung an die medizinische Vorrichtung geliefert wird, über die gesamte Verwendung der medizinischen Vorrichtung hinweg;
oder eine beliebige Kombination davon.

14. System der medizinischen Vorrichtung nach Anspruch 13, wobei die Energiequellenspeicherkomponente ferner Anweisungen umfasst, die, wenn sie durch die Energiequellenprozessoreinheit ausgeführt werden, die Energiequellenrechenvorrichtung veranlassen zum:
Empfangen, von dem Netzwerkserver der medizinischen Vorrichtung, von Werten des einen oder der mehreren zusätzlichen Indikatoren, die jedem der Identitätscodes der medizinischen Vorrichtung in der Datenbank der medizinischen Vorrichtung entsprechen; und
Steuern der Funktion der Energiequelle basierend auf dem Wert des einen oder der mehreren zusätzlicher Indikatoren, die dem Identitätscode der medizinischen Vorrichtung entsprechen, der gleich dem Identifikationscode ist.

15. System der medizinischen Vorrichtung nach Anspruch 13, wobei die Netzwerkserverspeicherkomponente Anweisungen umfasst, die, wenn sie durch die Netzwerkserverprozessoreinheit ausgeführt werden, die Netzwerkserverprozessoreinheit veranlassen zum:
Empfangen, von der Energiequelle der medizinischen Vorrichtung, von Werten des einen oder der mehreren zusätzlichen Indikatoren, die jedem der Identitätscodes der medizinischen Vorrichtung in der Datenbank der medizinischen Vorrichtung entsprechen; und
Aktualisieren der Werte des einen oder der mehreren zusätzlichen Indikatoren, die jedem der Identitätscodes der medizinischen Vorrichtung in der Datenbank der medizinischen Vorrichtung entsprechen.

## Revendications

1. Source d'énergie de dispositif médical (220b), comprenant :
une source d'énergie (435) ;
une interface de puissance de source d'énergie (226b) configurée pour libérer de l'énergie électrique de la source d'énergie (435) ; et
un dispositif informatique de source d'énergie (450), comprenant :
une unité de processeur de source d'énergie (410) ;
un composant de stockage de mémoire de source d'énergie (412) en communication fonctionnelle avec l'unité de processeur de source d'énergie ;
une interface de communication de réseau de source d'énergie (228) en communication fonctionnelle avec l'unité de processeur de source d'énergie ; et
une interface de données de source d'énergie (224) en communication fonctionnelle avec l'unité de processeur de source d'énergie,
dans laquelle le dispositif informatique de source d'énergie (450) est configuré pour commander une fonction de la source d'énergie, et
dans laquelle, le composant de stockage de mémoire de source d'énergie (412) comprend des instructions qui, lorsqu'elles sont exécutées par l'unité de processeur de source d'énergie, amènent le dispositif informatique de source d'énergie à :
recevoir un code d'identification par le biais de l'interface de données de source d'énergie ;
recevoir une pluralité de codes d'identité de dispositif médical par le biais de l'interface de communication de réseau de source d'énergie ;
comparer le code d'identification à chacun de la pluralité de codes d'identité de dispositif médical ; et
commander la fonction de la source d'énergie en fonction de la comparaison du code d'identification à chacun de la pluralité de codes d'identité de dispositif médical.

2. Source d'énergie de dispositif médical selon la revendication 1, dans laquelle le code d'identification comprend deux chaînes
d'identification (610x, 610x'), chacune des deux chaînes d'identification comprenant une chaîne de caractères lisibles par processeur.

3. Source d'énergie de dispositif médical selon la revendication 2, dans laquelle les instructions qui amènent le dispositif informatique de source d'énergie à comparer le code d'identification à chacun de la pluralité de codes d'identité de dispositif médical comprennent des instructions qui amènent le dispositif informatique de source d'énergie à comparer chacune des deux chaînes d'identification à chacune des deux chaînes d'identité comprenant chacun des codes d'identité de dispositif médical.

4. Source d'énergie de dispositif médical selon la revendication 1, dans laquelle, le composant de stockage de mémoire de source d'énergie comprend des instructions qui, lorsqu'elles sont exécutées par l'unité de processeur de source d'énergie, amènent en outre le dispositif informatique de source d'énergie à recevoir, par le biais de l'interface de communication de réseau de source d'énergie, une pluralité d'indicateurs d'état de dispositif médical, dans laquelle chaque indicateur d'état de dispositif médical correspondant à chacun de la pluralité de codes d'identité de dispositif médical,
dans laquelle éventuellement les instructions qui amènent le dispositif informatique de source d'énergie à commander la fonction de la source d'énergie comprennent en outre des instructions qui amènent le dispositif informatique de source d'énergie à commander la fonction de la source d'énergie en fonction des indicateurs d'état de dispositif médical correspondant à un code d'identité de dispositif médical égal au code d'identification.

5. Source d'énergie de dispositif médical selon la revendication 1, dans laquelle le composant de stockage de mémoire de source d'énergie comprend des instructions qui, lorsqu'elles sont exécutées par l'unité de processeur de source d'énergie, amènent en outre le dispositif informatique de source d'énergie à conserver, dans le composant de stockage de mémoire de source d'énergie :
une valeur d'électrification correspondant à une quantité d'énergie fournie par la source d'énergie ;
une valeur de temps d'électrification correspondant à une durée pendant laquelle la source d'énergie fournit une quantité d'énergie ;
un nombre d'électrification correspondant au nombre de fois où la source d'énergie fournit une quantité d'énergie ;
ou des combinaisons de ceux-ci.

6. Source d'énergie de dispositif médical selon la revendication 1, comprenant en outre un affichage utilisateur en communication fonctionnelle avec l'unité de processeur de source d'énergie.

7. Système de dispositif médical, comprenant :
un dispositif médical (210b) comprenant :
un composant de stockage de mémoire de dispositif (312) configuré pour stocker un code d'identification ;
une interface de données de dispositif (218) en connexion fonctionnelle avec le composant de stockage de mémoire ; et
une interface de puissance de dispositif (217b) configurée pour recevoir une puissance électrique d'une source d'énergie (435) ;
source d'énergie de dispositif médical (220b), comprenant :
la source d'énergie (435) ;
une interface de puissance de source d'énergie (226b) en communication fonctionnelle avec l'interface de puissance de dispositif et configurée pour libérer de l'énergie électrique de la source d'énergie au dispositif médical ; et
un dispositif informatique de source d'énergie (450), comprenant :
une unité de processeur de source d'énergie (410) ;
un composant de stockage de mémoire de source d'énergie (412) en communication fonctionnelle avec l'unité de processeur de source d'énergie ;
une interface de communication de réseau de source d'énergie (228) en communication fonctionnelle avec l'unité de processeur de source d'énergie et configurée pour transmettre des données à un réseau de communication et recevoir des données de celui-ci ; et
une interface de données de source d'énergie (224) en connexion fonctionnelle avec l'interface de données de dispositif,
dans lequel le dispositif informatique de source d'énergie est configuré pour commander une fonction de la source d'énergie ; et
un serveur de réseau de dispositif médical (230), comprenant :
une unité de processeur de serveur de réseau (510) ;
un composant de stockage de mémoire de serveur de réseau (512) en communication fonctionnelle avec l'unité de processeur de serveur de réseau et configuré pour stocker une base de données de dispositif médical comprenant une pluralité de codes d'identité de dispositif médical et d'indicateurs d'état de dispositif médical correspondants ; et
une interface de communication de serveur de réseau (232) en communication fonctionnelle avec l'unité de processeur de serveur de réseau et configurée pour transmettre des données à au moins une source de puissance de dispositif médical et recevoir des données de celui-ci par le biais du réseau de communication ; dans lequel, le composant de stockage de mémoire de source d'énergie comprend des instructions qui, lorsqu'elles sont exécutées par l'unité de processeur de source d'énergie, amènent le dispositif informatique de source d'énergie à :
recevoir, du composant de stockage de mémoire de dispositif, le code d'identification ;
recevoir, du composant de stockage de mémoire de serveur de réseau, la pluralité de codes d'identité de dispositif médical de la base de données de dispositif médical ;
comparer le code d'identification à chacun de la pluralité de codes d'identité de dispositif médical ; et
commander la fonction de la source d'énergie en fonction de la comparaison de l'au moins un code d'identification à la pluralité de codes d'identité de dispositif médical.

8. Système de dispositif médical selon la revendication 7, dans lequel le code d'identification comprend deux chaînes d'identification, chacune des deux chaînes d'identification (610x, 610x') comprenant une chaîne de caractères lisibles par processeur, et
dans lequel les instructions qui amènent le dispositif informatique de source d'énergie à comparer le code d'identification à chacun de la pluralité de codes d'identité de dispositif médical comprennent des instructions qui amènent le dispositif informatique de source d'énergie à comparer chacune des deux chaînes d'identification à chacune des deux chaînes d'identité comprenant chacun des codes d'identité de dispositif médical.

9. Système de dispositif médical selon la revendication 7, dans lequel, le composant de stockage de mémoire de source d'énergie comprend des instructions qui, lorsqu'elles sont exécutées par l'unité de processeur de source d'énergie, amènent en outre le dispositif informatique de source d'énergie à recevoir, par le biais de l'interface de communication de réseau de source d'énergie, une pluralité d'indicateurs d'état de dispositif médical, dans lequel chacun de la pluralité d'indicateurs d'état de dispositif médical correspond à chacun de la pluralité de codes d'identité de dispositif médical, et
dans lequel les instructions qui amènent le dispositif informatique de source d'énergie à commander la fonction de la source d'énergie comprennent en outre des instructions qui amènent le dispositif informatique de source d'énergie à commander la fonction de la source d'énergie en fonction d'un indicateur d'état de dispositif médical correspondant à un code d'identité de dispositif médical égal au code d'identification.

10. Système de dispositif médical selon la revendication 7, dans lequel le composant de stockage de mémoire de source d'énergie comprend en outre des instructions qui, lorsqu'elles sont exécutées par l'unité de processeur de source d'énergie, amènent le dispositif informatique de source d'énergie à transmettre, au serveur de réseau de dispositif médical, des données pour mettre à jour un indicateur d'état de dispositif médical correspondant à un code d'identité de dispositif médical égal au code d'identification.

11. Système de dispositif médical selon la revendication 10, dans lequel le composant de stockage de mémoire de serveur de réseau comprend des instructions qui, lorsqu'elles sont exécutées par l'unité de processeur de serveur de réseau, amènent l'unité de processeur de serveur de réseau à :
recevoir, de la source d'énergie de dispositif médical, des données pour mettre à jour un indicateur d'état de dispositif médical correspondant au code d'identité de dispositif médical égal au code d'identification ; et
mettre à jour l'indicateur d'état dans la base de données correspondant au code d'identité de dispositif médical égal au code d'identification.

12. Système de dispositif médical selon la revendication 7, dans lequel la base de données de dispositif médical comprend en outre un ou plusieurs indicateurs supplémentaires correspondant à chacun des codes d'identité de dispositif médical dans la base de données de dispositif médical.

13. Système de dispositif médical selon la revendication 7, dans lequel le composant de stockage de mémoire de source d'énergie comprend en outre des instructions qui, lorsqu'elles sont exécutées par l'unité de processeur de source d'énergie, amènent le dispositif informatique de source d'énergie à stocker dans le composant de stockage de mémoire de source d'énergie :
un indicateur des utilisations totales de dispositif médical ;
un indicateur, pour chaque utilisation des utilisations totales de dispositif médical, de :
une quantité de puissance fournie par la source d'énergie de dispositif médical au dispositif médical ; et
une durée pendant laquelle la source d'énergie de dispositif médical fournit la quantité d'énergie au dispositif médical ;
une quantité totale de puissance fournie par la source d'énergie de dispositif médical au dispositif médical sur les utilisations totales de dispositif médical ;
ou une quelconque combinaison de ceux-ci.

14. Système de dispositif médical selon la revendication 13, dans lequel le composant de stockage de mémoire de source d'énergie comprend en outre des instructions qui, lorsqu'elles sont exécutées par l'unité de processeur de source d'énergie, amènent le dispositif informatique de source d'énergie à :
recevoir, du serveur de réseau de dispositif médical, des valeurs de l'un ou plusieurs indicateurs supplémentaires correspondant à chacun des codes d'identité de dispositif médical dans la base de données de dispositif médical ; et
commander la fonction de la source d'énergie en fonction de la valeur de l'indicateur ou des indicateurs supplémentaires correspondant au code d'identité de dispositif médical égal au code d'identification.

15. Système de dispositif médical selon la revendication 13, dans lequel le composant de stockage de mémoire de serveur de réseau comprend des instructions qui, lorsqu'elles sont exécutées par l'unité de processeur de serveur de réseau, amènent l'unité de processeur de serveur de réseau à :
recevoir, de la source d'énergie de dispositif médical, des valeurs de l'indicateur ou des indicateurs supplémentaires correspondant à chacun des codes d'identité de dispositif médical dans la base de données de dispositif médical ; et
mettre à jour les valeurs de l'indicateur ou des indicateurs supplémentaires correspondant à chacun des codes d'identité de dispositif médical dans la base de données de dispositif médical.
